# EUROPEAN PATENT SPECIFICATION

(11) **EP 4 588 436 B1**
(45) Date of publication and mention of the grant of the patent: **09.12.2026**
(21) Application number: 23865615.1
(22) Date of filing: 15.09.2023
(51) Int. Cl.: A61B 5/11, A61B 5/22, A61B 5/397, A61B 5/00

(54) **METHOD AND DEVICE FOR EVALUATING EATING ABILITY**
VERFAHREN UND VORRICHTUNG ZUR BEURTEILUNG DER ESSFÄHIGKEIT
PROCÉDÉ ET DISPOSITIF D'ÉVALUATION DE LA CAPACITÉ D'ALIMENTATION

(30) Priority: 16.09.2022 JP 2022148634
(43) Date of publication of application: 23.07.2025
(73) Proprietor: MEIJI CO., LTD, Chuo-ku Tokyo 104-8306 (JP)
(72) Inventor: HISAJIMA, Tomoko, Hachioji-shi, Tokyo 192-0919 (JP); KUBOTA, Takayuki, Hachioji-shi, Tokyo 192-0919 (JP)
(74) Representative: TBK
(86) International application number: PCT/JP2023/033681
(87) International publication number: WO 2024/058266

(56) References cited:
- JP-A- 2012 139 442
- JP-A- 2019 067 302
- JP-A- 2020 148 692
- JP-A- 2021 154 064
- US-A1- 2002 133 194
- UEHARA FUMIKO ET AL: "Differentiation of Feeding Behaviors Based on Masseter and Supra-Hyoid Muscle Activity", FRONTIERS IN PHYSIOLOGY, vol. 11, 12 June 2020 (2020-06-12), CH, pages 1 - 8, XP093303522, ISSN: 1664-042X, DOI: 10.3389/fphys.2020.00618
- FUEKI K ET AL: "Comparison of electromyographic activity of jaw-closing muscles between mixing ability test and masticatory performance test", JOURNAL OF PROSTHODONTIC RESEARCH, ELSEVIER, AMSTERDAM, NL, vol. 53, no. 2, 1 April 2009 (2009-04-01), pages 72 - 77, XP025980098, ISSN: 1883-1958, [retrieved on 20081227], DOI: 10.1016/J.JPOR.2008.09.003
- FURUYAMA TOMONARI: "Masticatory Performance in Elderly by Evaluating the Swallowing Behavior", NIHON HOTETSU SHIKA GAKKAI ZASSHI - JOURNAL OF THE JAPANPROSTHODONTIC SOCIETY, NIHON HOTETSU SHIKA GAKKAI, TOKYO, JP, vol. 44, no. 2, 1 January 2000 (2000-01-01), JP , pages 300 - 309, XP093146281, ISSN: 0389-5386, DOI: 10.2186/jjps.44.300
- SASAKURA CHE, TADAO FUKUI, ISAO SAITO: "A Feature of the Deglutition Muscle Activity Form in Patients with Mandibular Prognathism", THE JAPANESE JOURNAL OF JAW DEFORMITIES, vol. 20, no. 4, 1 December 2010 (2010-12-01), pages 283 - 291, XP093146283

## Description

### TECHNICAL FIELD

The present invention relates to an eating ability evaluation method and an eating ability evaluation device.

### BACKGROUND ART

With the advancement of a super aging society, development of a method of evaluating a mastication movement such as an eating ability has been advanced in order to improve quality of mastication movement of elderly people and solve problems related to mastication.

In order to evaluate a series of activities from a moment at which food is put into the mouth to a moment at which the food is swallowed, physiological evaluation methods using myoelectric potential measurement have been developed.

Patent Literature 1 discloses a method of evaluating the behavior of food in the mouth using a result of measuring a time-dynamic change in surface myoelectric potentials of left and right masseter muscles and suprahyoid muscles.

Patent Literature 2 discloses a physiological evaluation device for a texture capable of appropriately and objectively performing complicated food sensory evaluation by acquiring amounts of muscular activities correlated with amounts of work of each muscle from electromyograms of left and right masseter muscles and suprahyoid muscles and obtaining differences in the amounts of muscular activities depending on food and differences in mastication periods.

### CITATION LIST

### PATENT LITERATURE

Patent Literature 1: Japanese Patent Application Publication No. 2020-148692
Patent Literature 2: Japanese Patent Application Publication No. 2012-139442

### NON-PATENT LITERATURE

Non-Patent Literature 1: UEHARA FUMIKO ET AL: "Differentiation of Feeding Behaviors Based on Masseter and Supra-Hyoid Muscle Activity", FRONTIERS IN PHYSIOLOGY, vol. 11, 12 June 2020 (2020-06-12), pages 1-8, XP093303522, CH ISSN: 1664-042X, DOI: 10.3389/fphys.2020.00618
Non-Patent Literature 2: FUEKI K ET AL: "Comparison of electromyographic activity of jaw-closing muscles between mixing ability test and masticatory performance test", JOURNAL OF PROSTHODONTIC RESEARCH, ELSEVIER, AMSTERDAM, NL, vol. 53, no. 2, 1 April 2009 (2009-04-01), pages 72-77, XP025980098, ISSN: 1883-1958, DOI: 10.1016/J.JPOR.2008.09.003

### SUMMARY OF INVENTION

### PROBLEMS TO BE SOLVED BY THE INVENTION

Although measuring the amount (µV·s) of work of each muscle by acquiring electromyograms of left and right masseter muscles and suprahyoid muscles and calculating areas of electromyogram waveforms from action potentials (µV) and action times (s) of the muscles in order to evaluate an eating ability is conceivable, it is difficult to perform comparison among values of the same human subject measured on different days and to perform comparison among human subjects while it is possible to perform comparison among food kinds in measurement performed one time for the same human subject.

Although normalizing (standardizing) measured values in order to perform comparison among values of the same human subject measured on different days and to perform comparison among human subjects is conceivable, there is a problem that it is difficult to measure the amount of work at the time of maximum occlusion in a case of a human subject who has a difficulty in following instructions, such as an elderly person or an infant, a case of a human subject who is suffering from a stomatognathic disease, or the like if measurement of the amount of work at the time of maximum occlusion using an electromyogram waveform at the time of a maximum load for the normalization (standardization) is attempted.

Therefore, it has been desired to enable comparison among values of the same human subject measured on different days and comparison among human subjects in a case where the amount of work of each muscle is measured to evaluate an eating ability.

Thus, the present invention provides an eating ability evaluation method and an eating ability evaluation device that enable comparison among values of the same human subject measured on different days and comparison among human subjects when the amount of work of each of left and right masseter muscles and suprahyoid muscles is measured to evaluate an eating ability.

The invention is defined in the appended set of claims.

### MEANS FOR SOLVING THE PROBLEMS

An eating ability evaluation method according to the present invention is defined in claims 1 to 12. An eating ability evaluation device according to the present invention is defined in claim 13.

An eating ability evaluation method includes, an acquisition step of acquiring myoelectric potential data on a plurality of facial muscles when a subject person masticates, a calculation step of calculating a ratio of an amount of work of a first muscle, which is one of the plurality of facial muscles involved in mastication, to a total amount of work of all of the plurality of facial muscles involved in mastication for each unit time on the basis of the acquired myoelectric potential data, and an evaluation step of evaluating an eating ability of the subject person based on the ratio.

In one eating ability evaluation method, the plurality of muscles include a masseter muscle and suprahyoid muscles, and the first muscle is the masseter muscle.

In one eating ability evaluation method, the unit time is from a start of mastication to a timing immediately before swallowing.

In one eating ability evaluation method, in the acquisition step, motion analysis data for analyzing motion of the subject person when the myoelectric potential data is measured is acquired, and in the calculation step, the myoelectric potential data in a period during which the subject person is not masticating in the motion analysis data is excluded from a calculation target of the ratio.

In one eating ability evaluation method, the motion analysis data is video data of imaging the subject person when the myoelectric potential data is acquired.

In one eating ability evaluation method, the motion analysis data is recorded sound data of recording sound in surroundings of the subject person when the myoelectric potential data is acquired.

In one eating ability evaluation method, in the evaluation step, the eating ability is determined to be higher as a range between a maximum value and a minimum value of the ratio in a predetermined time becomes wider.

In one eating ability evaluation method, in the evaluation step, the eating ability is determined to be more excellent as a median of the ratio in a predetermined time becomes larger.

In one eating ability evaluation method, in the evaluation step, the eating ability is evaluated in accordance with a median and a range between a maximum value and a minimum value of the ratio in a predetermined time.

In one eating ability evaluation method, in the evaluation step, the eating ability is determined to be more excellent as an average value of the ratio in a predetermined time becomes larger.

In one eating ability evaluation method, in the evaluation step, the eating ability is determined to be more excellent as a mode of the ratio in a predetermined time becomes larger.

In one eating ability evaluation method, the myoelectric potential data is a value measured when the subject person masticates food that contains all of an ingredient with a first hardness, an ingredient with a second hardness that is higher than the first hardness, and an ingredient with a third hardness that is higher than the ingredient with the second hardness.

In one eating ability evaluation method, the myoelectric potential data is a value measured when the subject person masticates food that, from among an ingredient with a first hardness, an ingredient with a second hardness that is higher than the first hardness, and an ingredient with a third hardness that is higher than the ingredient with the second hardness, contains the ingredients with the first hardness and the third hardness and does not contain the ingredient with the second hardness.

In one eating ability evaluation method, the first hardness food is a food that can be crushed by a pressure of less than 1 N/cm², the second hardness food is a food that can be crushed by a pressure of 1 N/cm² or more and less than 30 N/cm², and the third hardness food is a food that can be crushed by a pressure of 30 N/cm² or more.

In one eating ability evaluation method, the plurality of muscles include left and right masseter muscles and suprahyoid muscles, and the amount of work of the first muscle is a total value of amounts of work of the left and right masseter muscles.

In one eating ability evaluation method, the plurality of muscles include any of left and right masseter muscles and suprahyoid muscles, and the first muscle is any of the left and right masseter muscles.

In one eating ability evaluation method, the plurality of muscles include zygomatic muscles and suprahyoid muscles, and the first muscle is the zygomatic muscles.

In one eating ability evaluation method, the plurality of muscles include temporalis muscles and suprahyoid muscles, and the first muscle is the temporalis muscles.

In one eating ability evaluation method, the plurality of muscles is a combination of one or more of left and right masseter muscles, and temporalis muscles in addition to suprahyoid muscles, and the amount of work of the first muscle is a total value of an amount of work of one kind of the left and right masseter muscles, and temporalis muscles or an amount of work of a combination thereof.

In one eating ability evaluation method, the evaluation step is performed using an evaluation model, the evaluation model is a model performing learning using myoelectric potential data for learning and eating ability data of a measuring person who has measured the myoelectric potential data for learning and generating a threshold value in the learning, and in the evaluation step, the eating ability of the subject person is output by inputting the ratio and comparing the generated threshold value with the ratio.

### ADVANTAGEOUS EFFECTS OF THE INVENTION

According to one disclosure, it is possible to perform comparison among values of the same human subject measured on different days and comparison among human subjects when the amount of work of each of left and right masseter muscles and suprahyoid muscles is measured to evaluate an eating ability.

### BRIEF DESCRIPTION OF DRAWINGS

[Fig. 1]

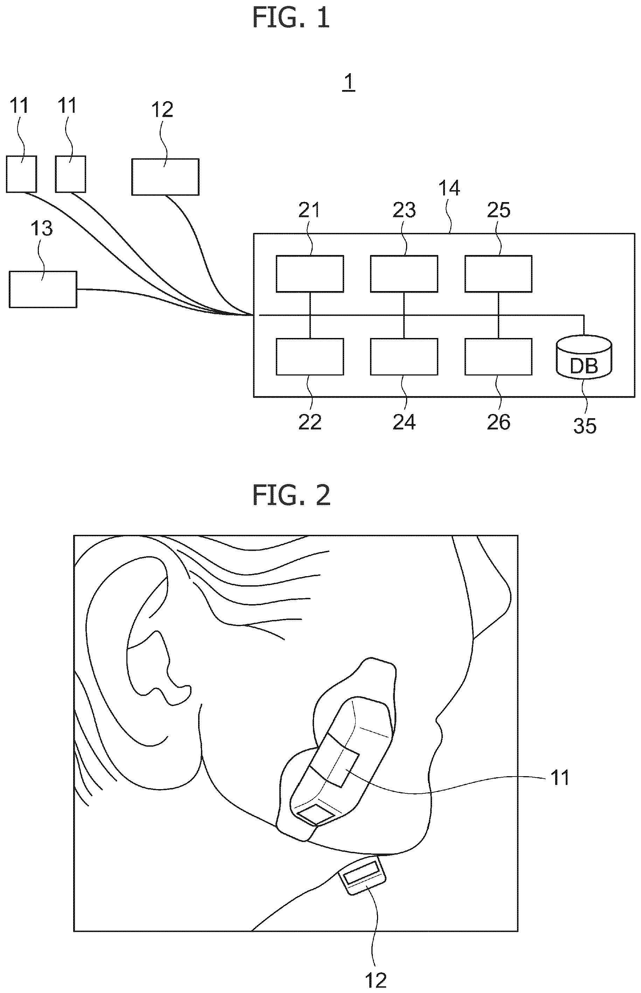

   Fig. 1 is a schematic diagram illustrating a configuration of an evaluation device according to an embodiment.
[Fig. 2]
   Fig. 2 is a schematic diagram for explaining a state where a myoelectric measurement unit for left and right masseter muscles and suprahyoid muscles in the evaluation device in Fig. 1 is attached to a human subject.
[Fig. 3]

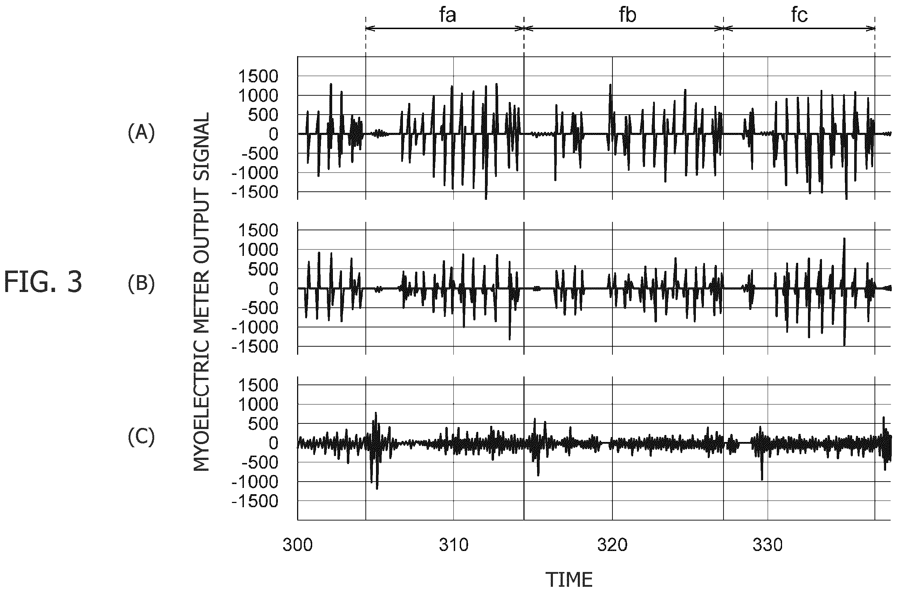

   Fig. 3 is an example of (A) an electromyogram of a right masseter muscle, (B) an electromyogram of a left masseter muscle, and (C) an electromyogram of suprahyoid muscles acquired in Example 1 in Fig. 1.
[Fig. 4A]

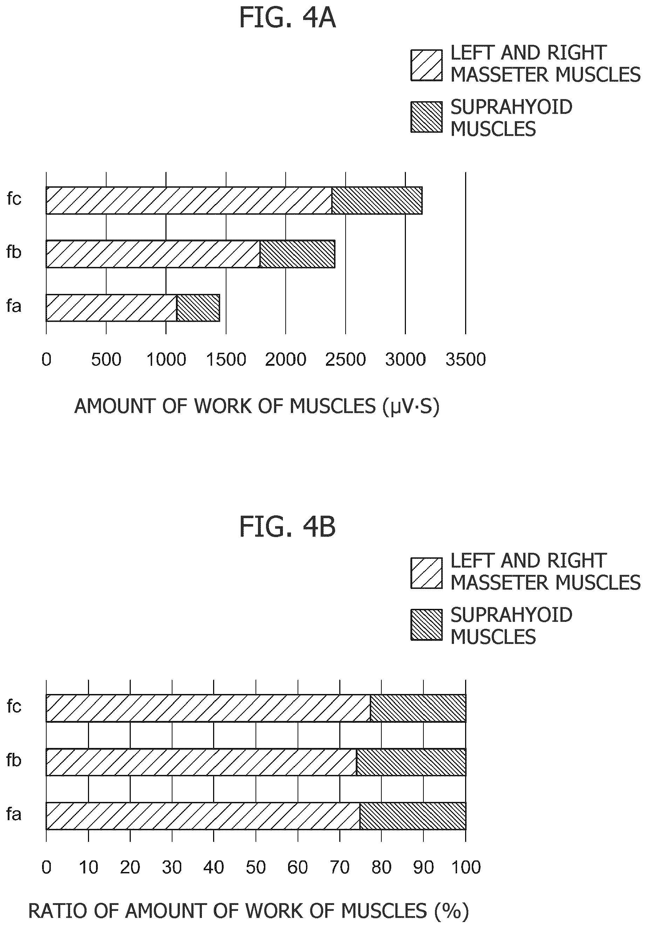

   Fig. 4A is an example of a graph indicating amounts of work of left and right masseter muscles and suprahyoid muscles in one masticating and swallowing motion of a human subject acquired in Example 1.
[Fig. 4B]
   Fig. 4B is an example of a graph indicating ratios of the amounts of work of left and right masseter muscles and suprahyoid muscles in one masticating and swallowing motion of a human subject acquired in Example 1.
[Fig. 5A]

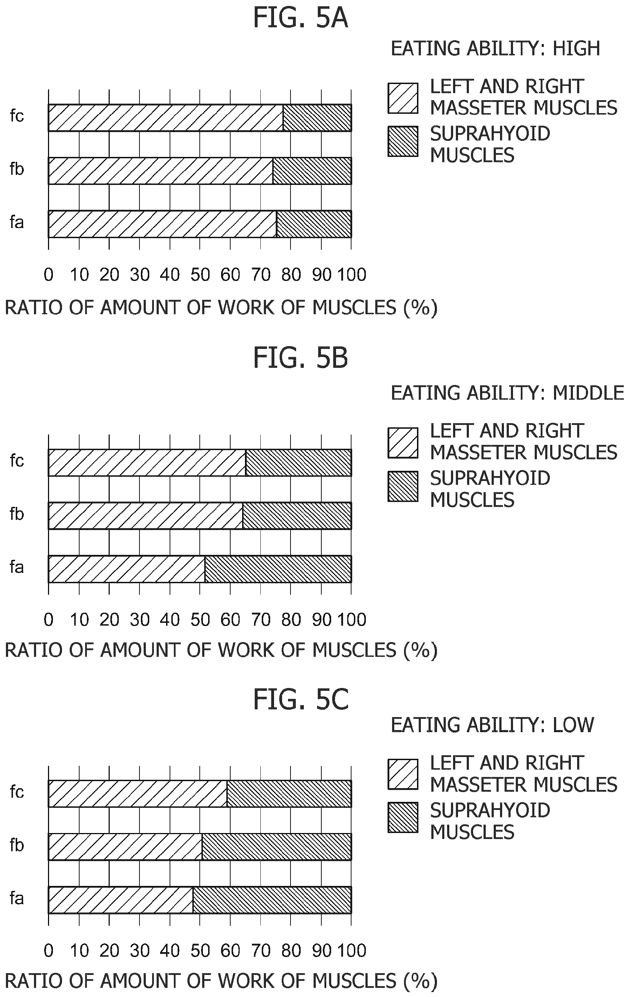

   Fig. 5A is a graph indicating ratios of the amounts of work of left and right masseter muscles and suprahyoid muscles in one masticating and swallowing motion of human subjects with high eating abilities acquired in Example 1.
[Fig. 5B]
   Fig. 5B is a graph indicating ratios of the amounts of work of left and right masseter muscles and suprahyoid muscles in one masticating and swallowing motion of human subjects with middle eating abilities acquired in Example 1.
[Fig. 5C]
   Fig. 5C is a graph indicating ratios of the amounts of work of left and right masseter muscles and suprahyoid muscles in one masticating and swallowing motion of human subjects with low eating abilities acquired in Example 1.
[Fig. 6A]

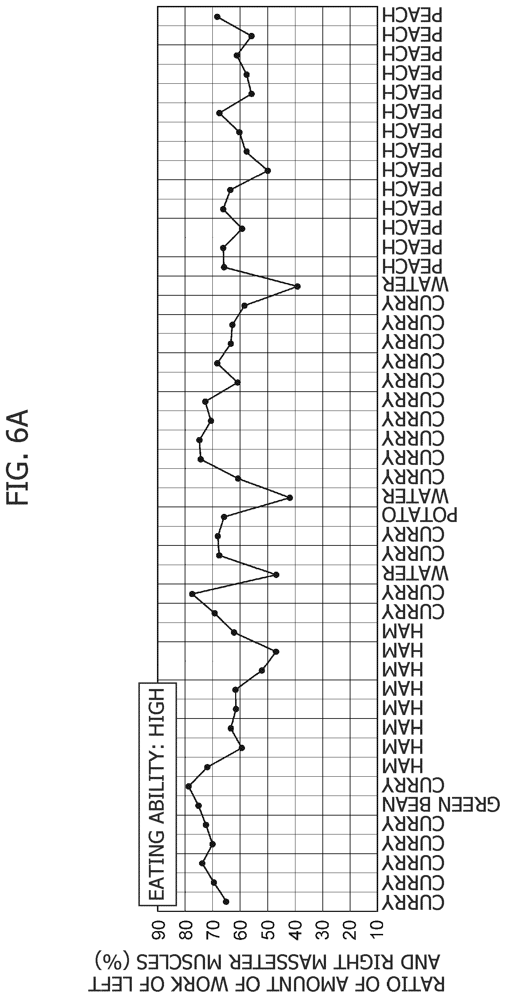

   Fig. 6A is a graph indicating, for each kind of taken food, ratios of the amounts of work of left and right masseter muscles in one masticating and swallowing motion of the human subjects with the high eating abilities acquired in Example 1.
[Fig. 6B]

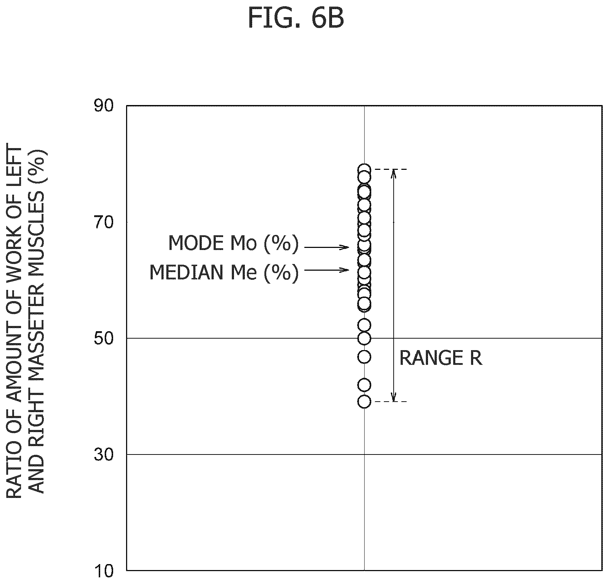

   Fig. 6B is a graph indicating ratios of the amounts of work of left and right masseter muscles in one masticating and swallowing motion of the human subjects with the high eating abilities acquired in Example 1.
[Fig. 6C]

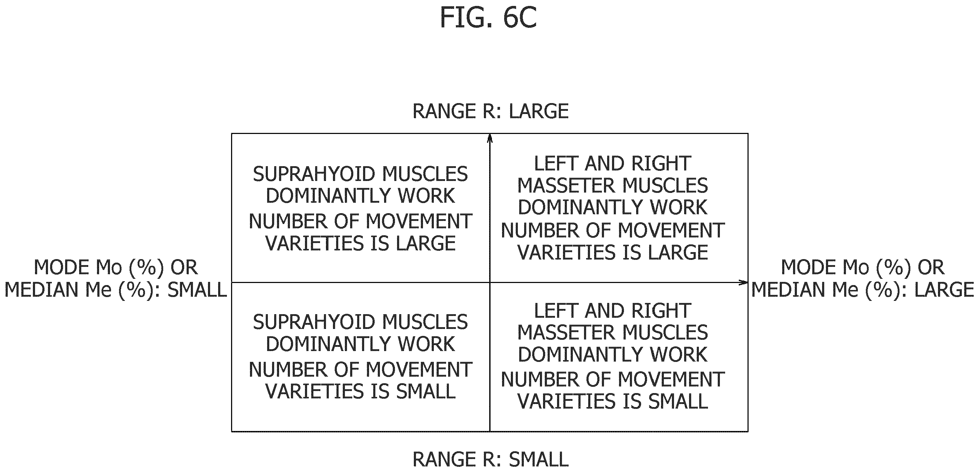

   Fig. 6C is a diagram in which the horizontal axis represents a mode or a median of ratios of the amounts of work of left and right masseter muscles and the vertical axis represents a range.
[Fig. 7A]

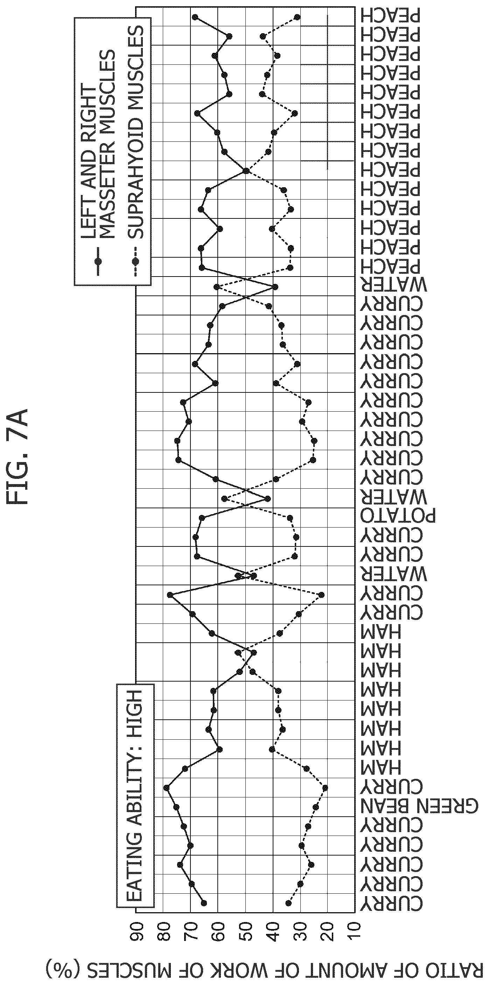

   Fig. 7A is a graph indicating, for each kind of taken food, ratios of the amounts of work of left and right masseter muscles and suprahyoid muscles in one masticating and swallowing motion of a human subject with a high eating ability acquired in Example 2.
[Fig. 7B]

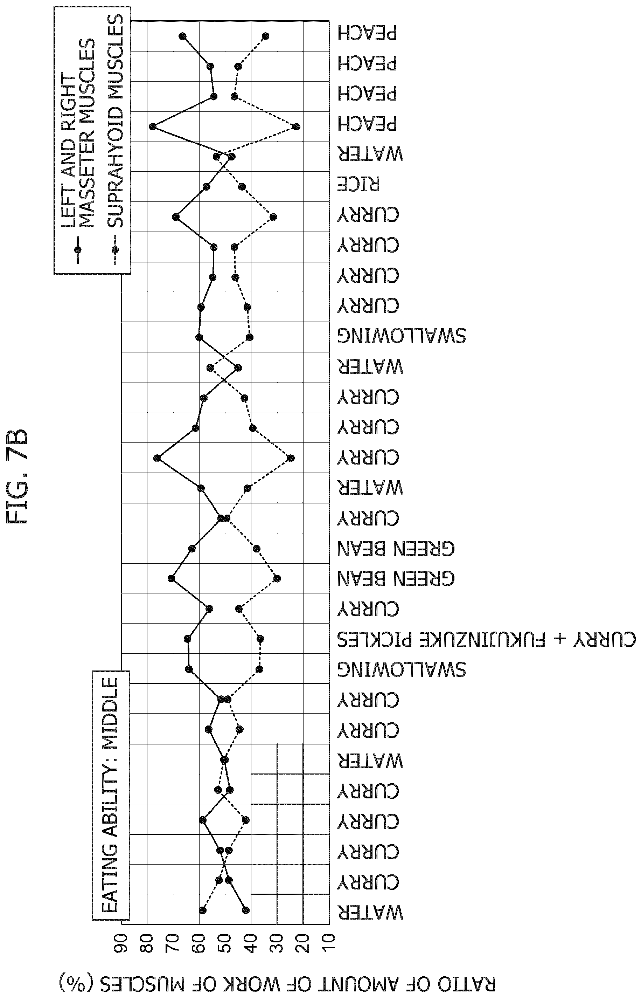

   Fig. 7B is a graph indicating, for each kind of taken food, ratios of the amounts of work of left and right masseter muscles and suprahyoid muscles in one masticating and swallowing motion of a human subject with a middle eating ability acquired in Example 2.
[Fig. 7C]

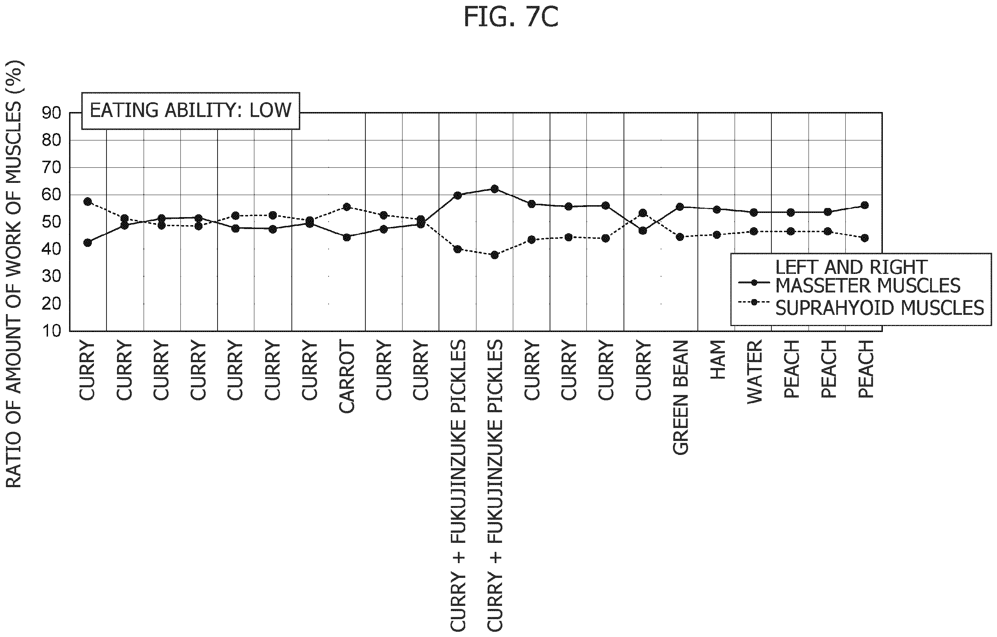

   Fig. 7C is a graph indicating, for each kind of taken food, ratios of the amounts of work of left and right masseter muscles and suprahyoid muscles in one masticating and swallowing motion of a human subject with a low eating ability acquired in Example 2.
[Fig. 7D]

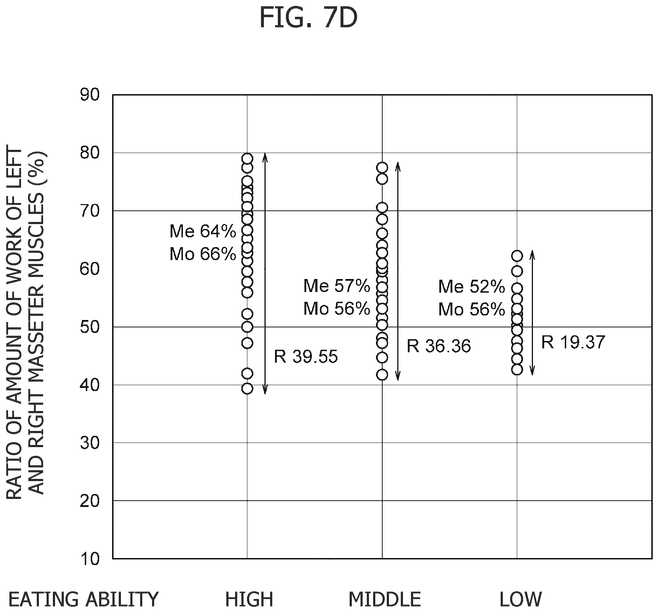

   Fig. 7D is a graph indicating ratios of the amounts of work of left and right masseter muscles in one masticating and swallowing motion of each of human subjects with high, middle, and low eating abilities acquired in Example 2.
[Fig. 7E]

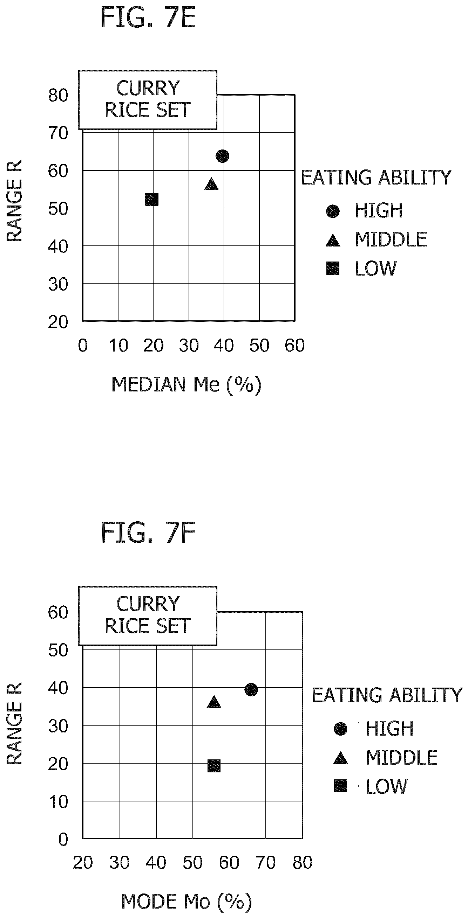

   Fig. 7E is a graph indicating a range with respect to a median of ratios of the amounts of work of left and right masseter muscles in Example 2.
[Fig. 7F]
   Fig. 7F is a graph indicating a range with respect to a mode of ratios of the amounts of work of left and right masseter muscles in Example 2.
[Fig. 8A]

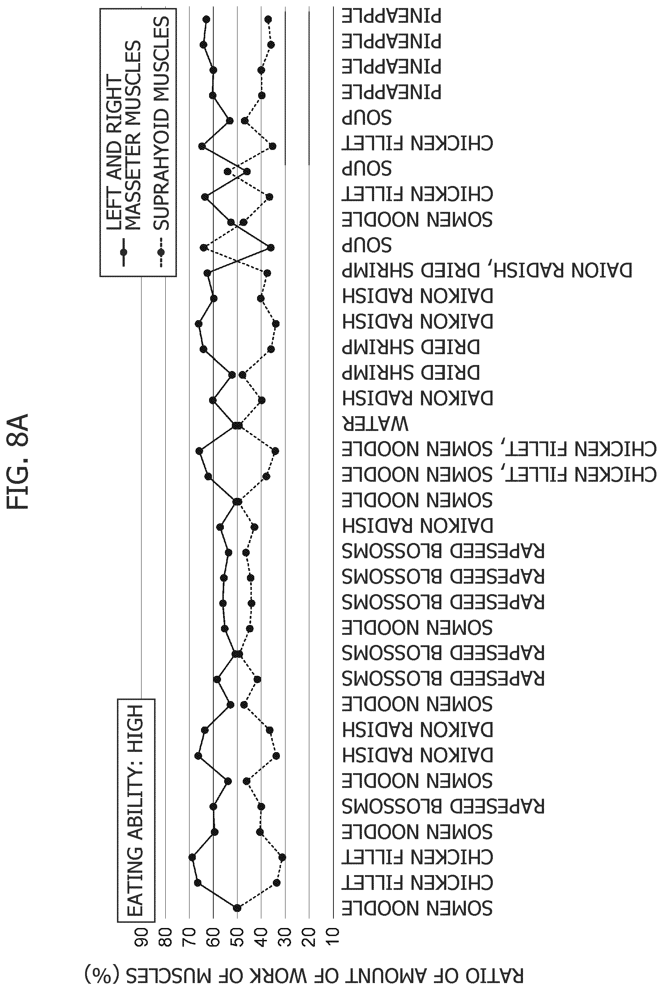

   Fig. 8A is a graph indicating, for each kind of taken food, ratios of the amounts of work of left and right masseter muscles and suprahyoid muscles on one masticating and swallowing motion of a human subject with a high eating ability acquired in Example 3.
[Fig. 8B]

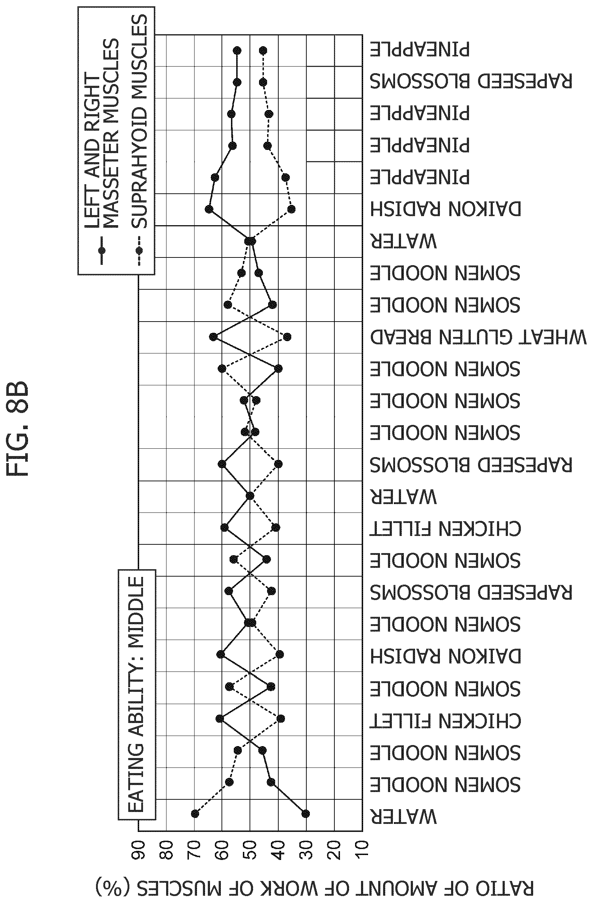

   Fig. 8B is a graph indicating, for each kind of taken food, ratios of the amounts of work of left and right masseter muscles and suprahyoid muscles in one masticating and swallowing motion of a human subject with a middle eating ability acquired in Example 3.
[Fig. 8C]

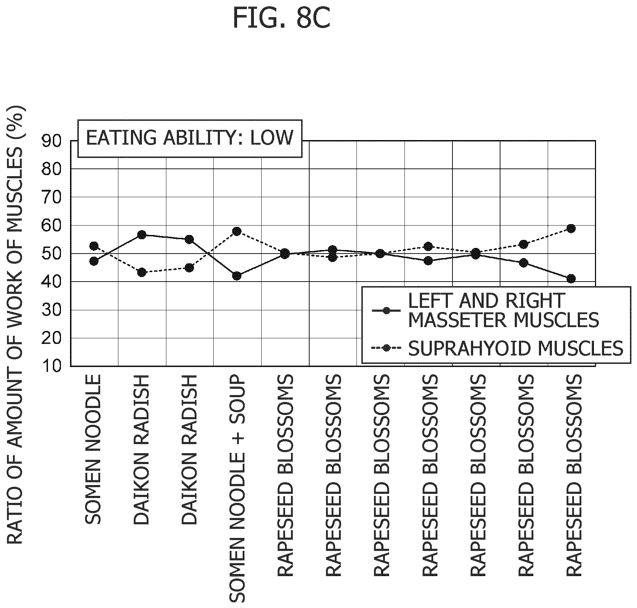

   Fig. 8C is a graph indicating, for each kind of taken food, ratios of the amounts of work of left and right masseter muscles and suprahyoid muscles in one masticating and swallowing motion of a human subject with a low eating ability acquired in Example 3.
[Fig. 8D]

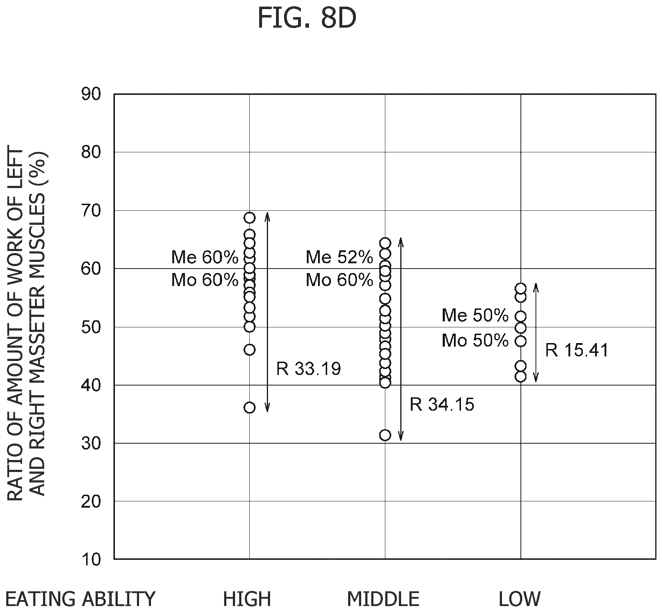

   Fig. 8D is a graph indicating ratios of the amounts of work of left and right masseter muscles in one masticating and swallowing motion of each of human subjects with high, middle, and low eating abilities acquired in Example 3.
[Fig. 8E]

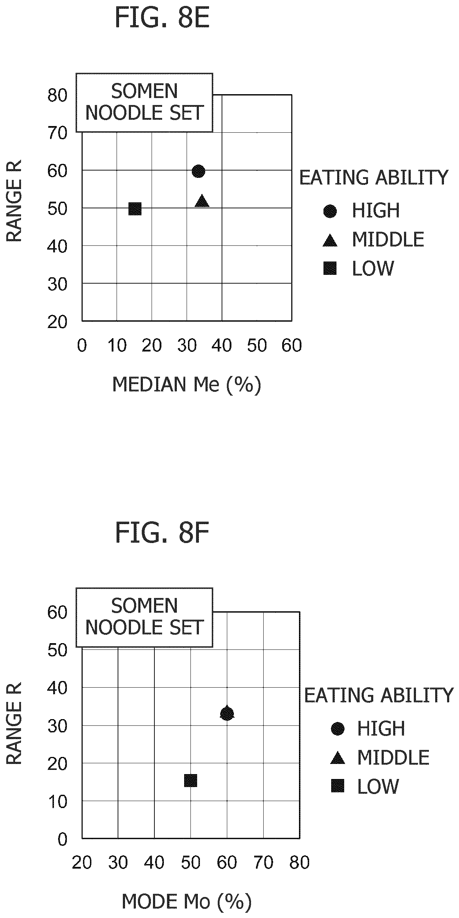

   Fig. 8E is a graph indicating a range with respect to a median of ratios of the amounts of work of left and right masseter muscles in Example 3.
[Fig. 8F]
   Fig. 8F is a graph indicating a range with respect to a mode of ratios of the amounts of work of left and right masseter muscles in Example 3.
[Fig. 9A]

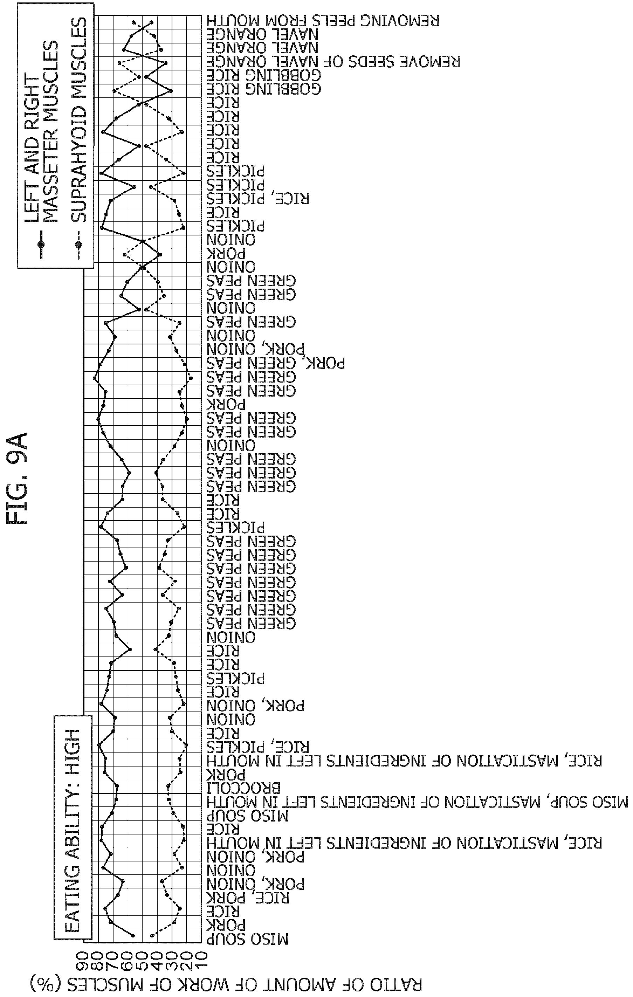

   Fig. 9A is a graph indicating, for each kind of taken food, ratios of the amounts of work of left and right masseter muscles and suprahyoid muscles in one masticating and swallowing motion of a human subject with a high eating ability acquired in Example 4.
[Fig. 9B]

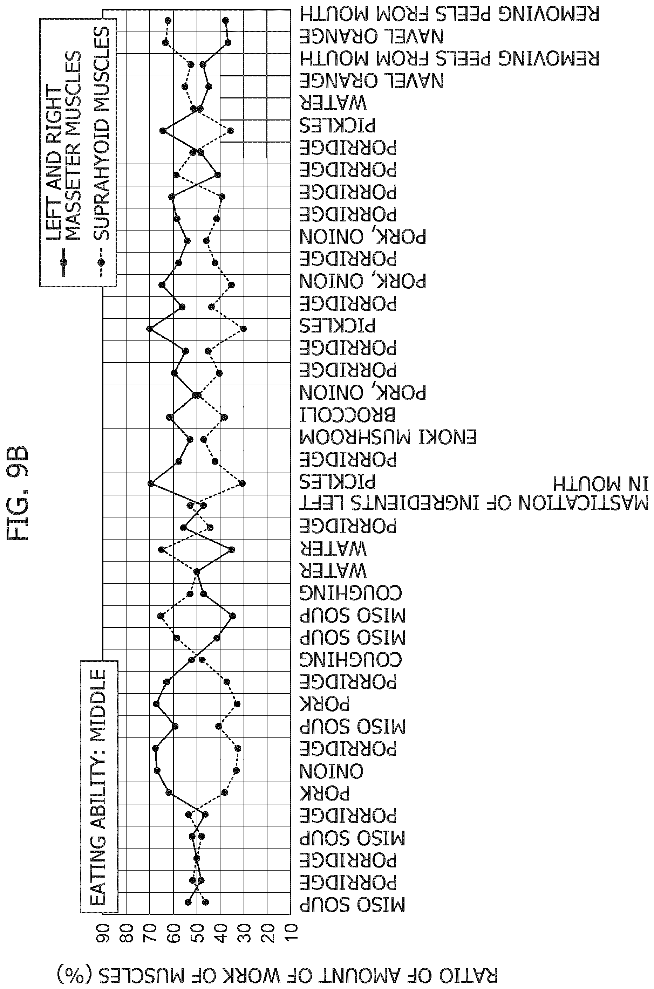

   Fig. 9B is a graph indicating, for each kind of taken food, ratios of the amounts of work of left and right masseter muscles and suprahyoid muscles in one masticating and swallowing motion of a human subject with a middle eating ability acquired in Example 4.
[Fig. 9C]

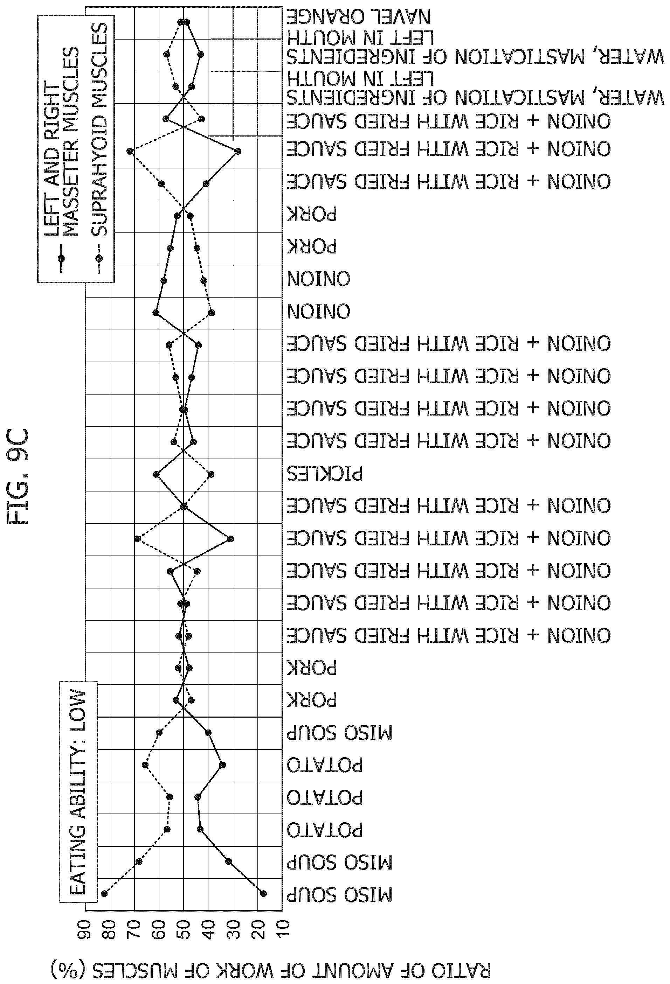

   Fig. 9C is a graph indicating, for each kind of taken food, ratios of the amounts of work of left and right masseter muscles and suprahyoid muscles in one masticating and swallowing motion of a human subject with a low eating ability acquired in Example 4.
[Fig. 9D]

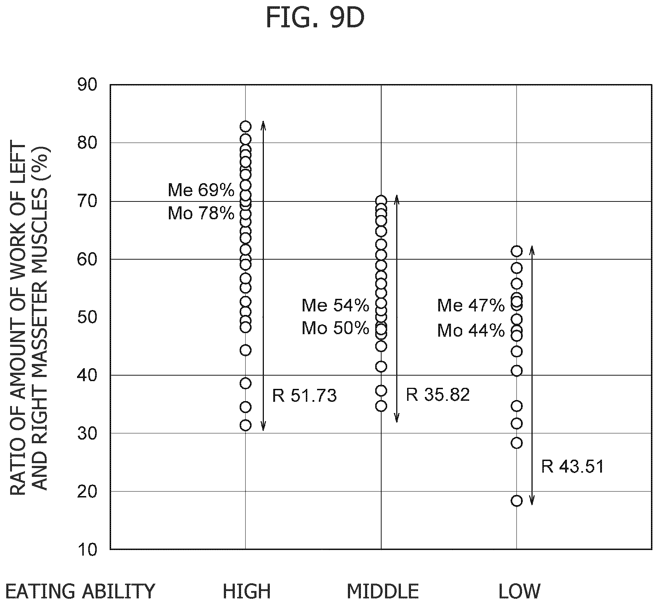

   Fig. 9D is a graph indicating ratios of the amounts of work of left and right masseter muscles in one masticating and swallowing motion of each of human subjects with high, middle, and low eating abilities acquired in Example 4.
[Fig. 9E]

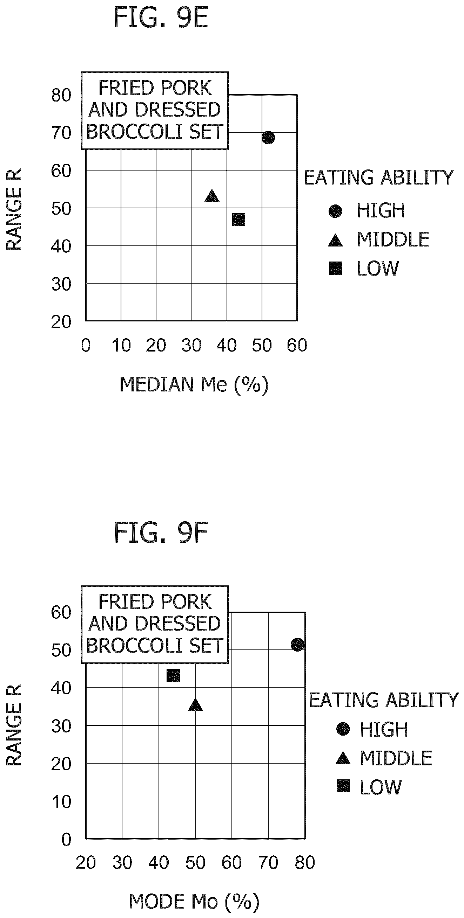

   Fig. 9E is a graph indicating a range with respect to a median of ratios of the amounts of work of left and right masseter muscles in Example 4.
[Fig. 9F]
   Fig. 9F is a graph indicating a range with respect to a mode of ratios of the amounts of work of left and right masseter muscles in Example 4.
[Fig. 10A]

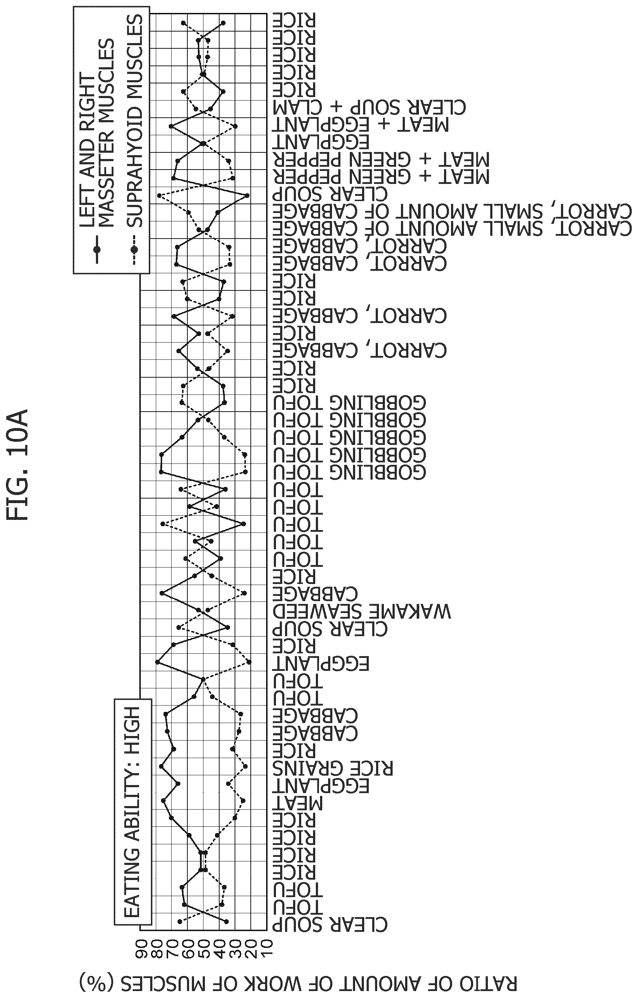

   Fig. 10A is a graph indicating, for each kind of taken food, ratios of the amounts of work of left and right masseter muscles and suprahyoid muscles in one masticating and swallowing motion of a human subject with a high eating ability acquired in Example 5.
[Fig. 10B]

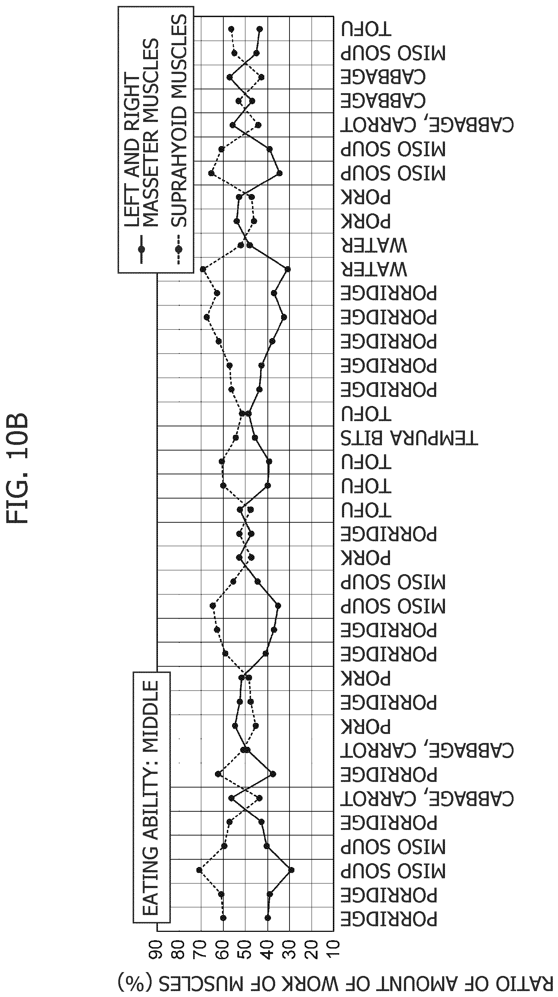

   Fig. 10B is a graph indicating, for each kind of taken food, ratios of the amounts of work of left and right masseter muscles and suprahyoid muscles in one masticating and swallowing motion of a human subject with a middle eating ability acquired in Example 5.
[Fig. 10C]

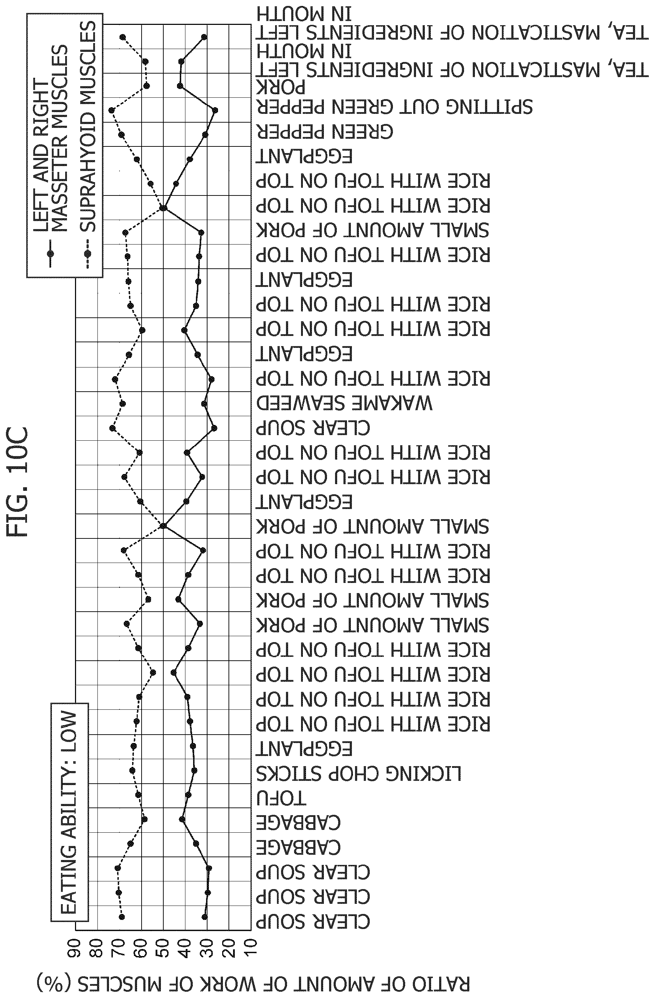

   Fig. 10C is a graph indicating, for each kind of taken food, ratios of the amounts of work of left and right masseter muscles and suprahyoid muscles in one masticating and swallowing motion of a human subject with a low eating ability acquired in Example 5.
[Fig. 10D]

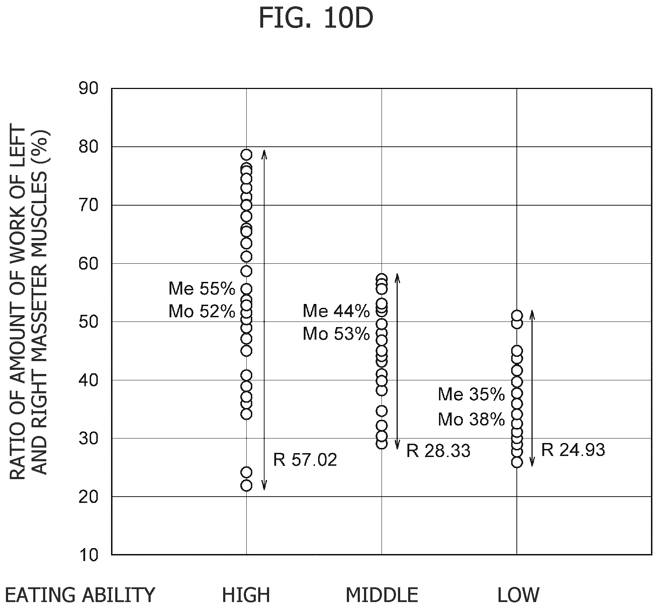

   Fig. 10D is a graph indicating ratios of the amounts of work of left and right masseter muscles in one masticating and swallowing motion of each of human subjects with high, middle, and low eating abilities acquired in Example 5.
[Fig. 10E]

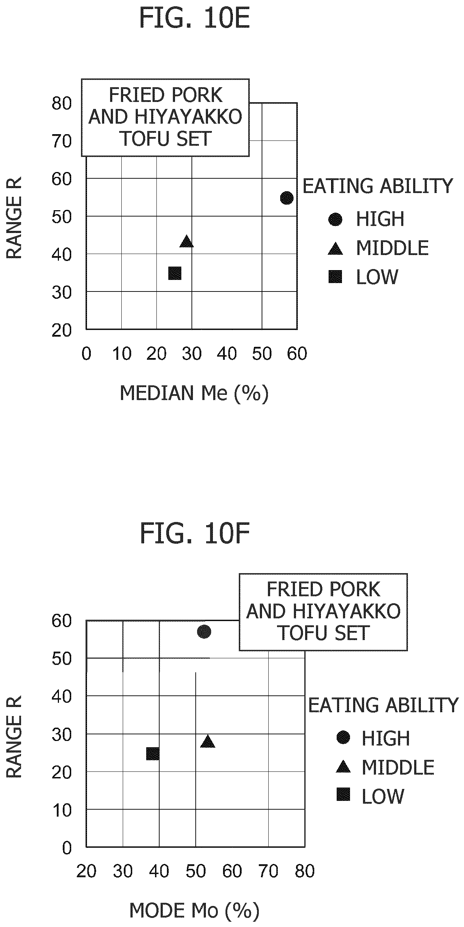

   Fig. 10E is a graph indicating a range with respect to a median of ratios of the amounts of work of left and right masseter muscles in Example 5.
[Fig. 10F]
   Fig. 10F is a graph indicating a range with respect to a mode of ratios of the amounts of work of left and right masseter muscles in Example 5.
[Fig. 11A]

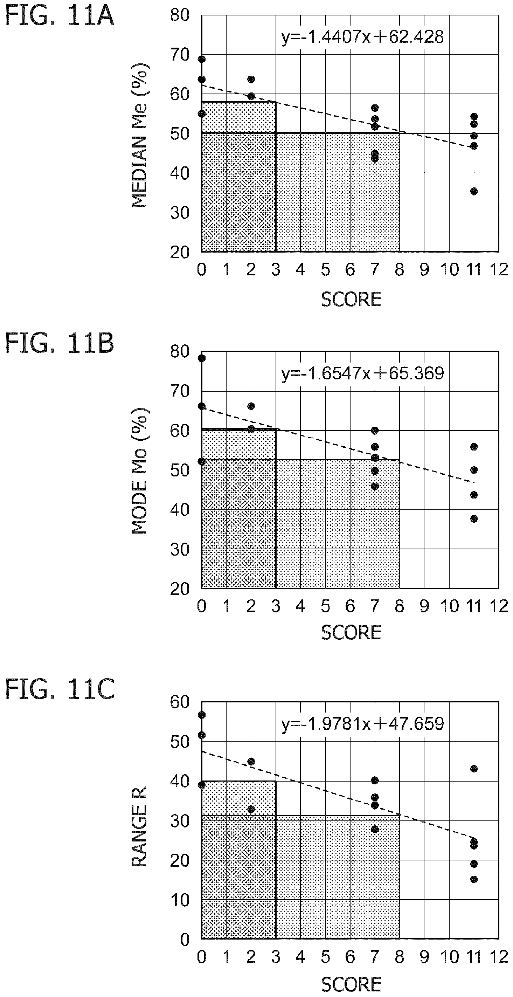

   Fig. 11A is a graph indicating medians of ratios of the amounts of work of left and right masseter muscles with respect to a test score of evaluation items related to an eating ability in Example 6.
[Fig. 11B]
   Fig. 11B is a graph indicating modes of ratios of the amounts of work of left and right masseter muscles with respect to a test score of evaluation items related to an eating ability in Example 6.
[Fig. 11C]
   Fig. 11C is a graph indicating a range of ratios of the amounts of work of left and right masseter muscles with respect to a test score of evaluation items related to an eating ability in Example 6.
[Fig. 11D]

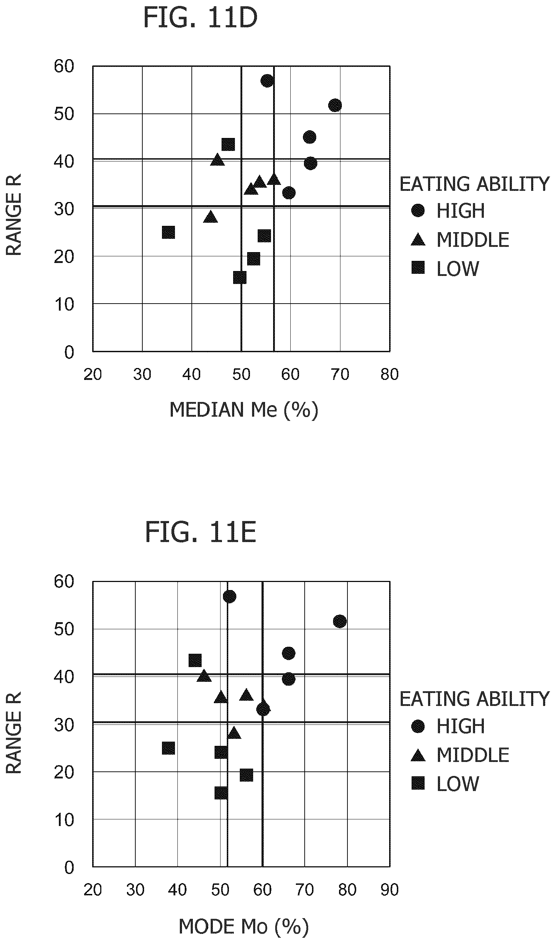

   Fig. 11D is a graph indicating the range with respect to the median of ratios of the amounts of work of left and right masseter muscles in Example 6.
[Fig. 11E]
   Fig. 11E is a graph indicating the range with respect to the mode of ratios of the amounts of work of left and right masseter muscles in Example 6.
[Fig. 12]

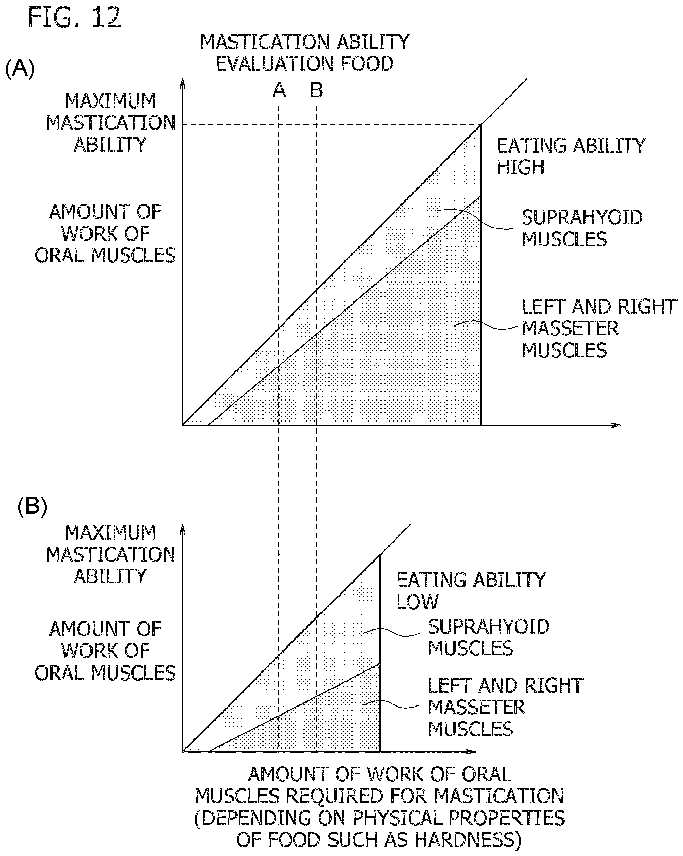

   Fig. 12 is a diagram for explaining ratios of the amounts of work of left and right masseter muscles of oral muscles and ratios of the amounts of work of suprahyoid muscles measured at the time of mastication with respect to the amount of work of oral muscles needed for mastication, where (A) illustrates a case of a human subject with a high eating ability, and (B) illustrates a case of a human subject with a low eating ability.
[Fig. 13]

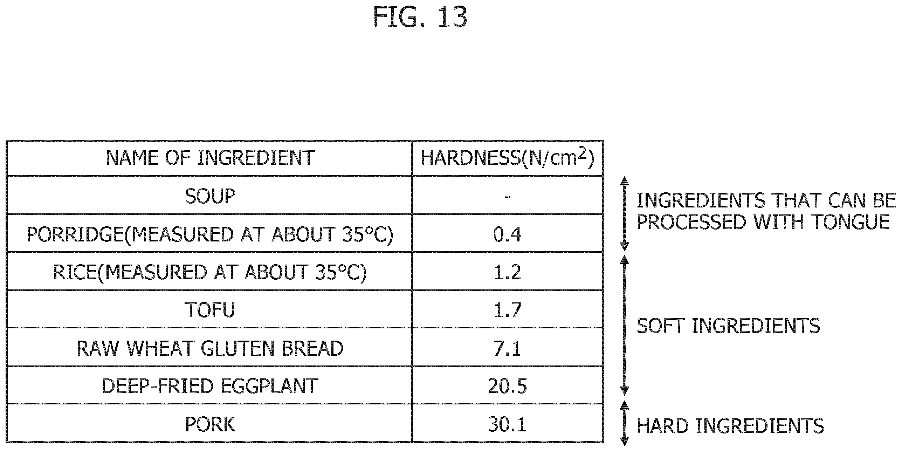

   Fig. 13 is a diagram illustrating an example of ingredients contained in a "pork and tofu set".
[Fig. 14]

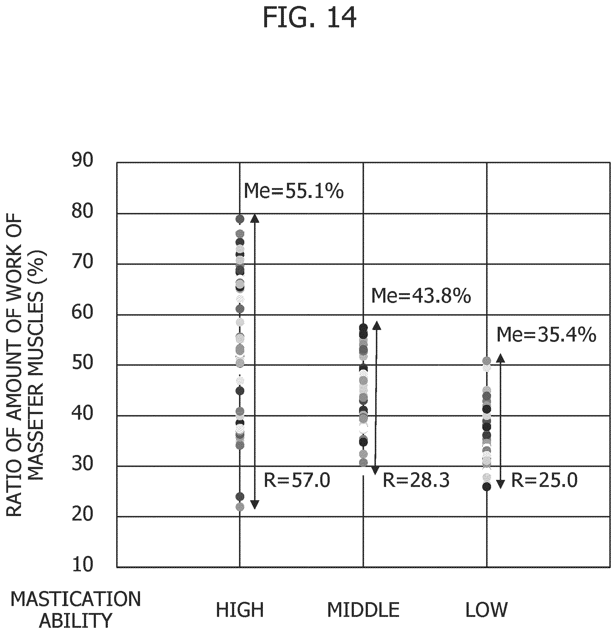

   Fig. 14 is a diagram illustrating an example of work amount ratios of masseter muscles for a food pattern 1 (containing all the ingredients).
[Fig. 15]

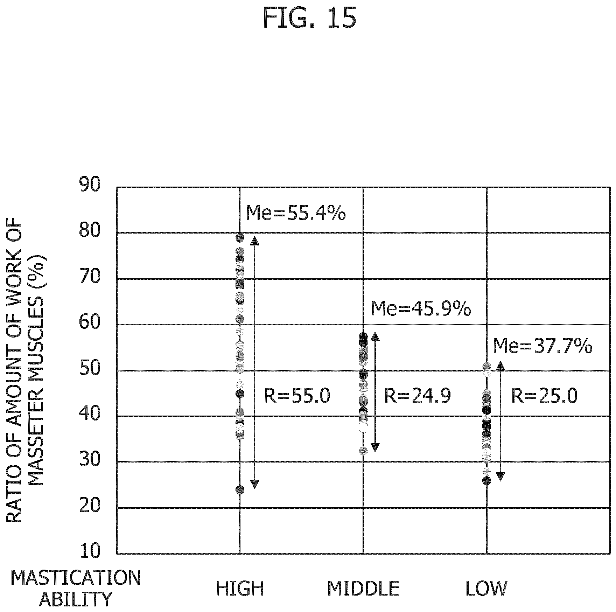

   Fig. 15 is a diagram illustrating an example of work amount ratios of masseter muscles for a food pattern 2 (excluding ingredients that can be processed by a tongue).
[Fig. 16]

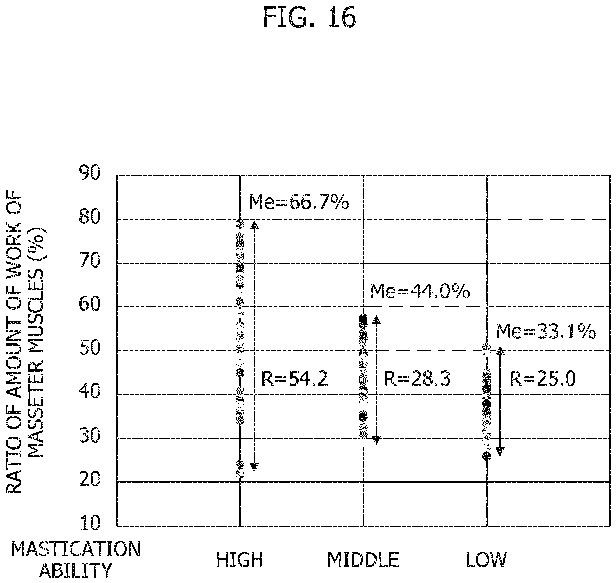

   Fig. 16 is a diagram illustrating an example of work amount ratios of masseter muscles for a food pattern 3 (excluding soft ingredients).
[Fig. 17]

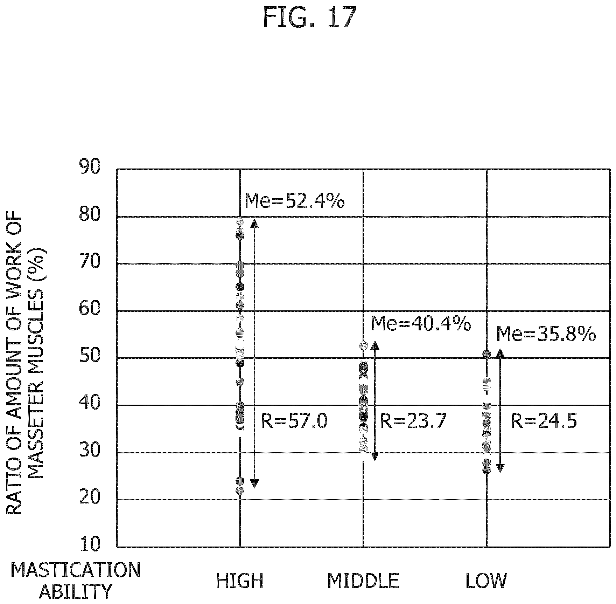

   Fig. 17 is a diagram illustrating an example of work amount ratios of masseter muscles for a food pattern 4 (excluding hard ingredients).
[Fig. 18]

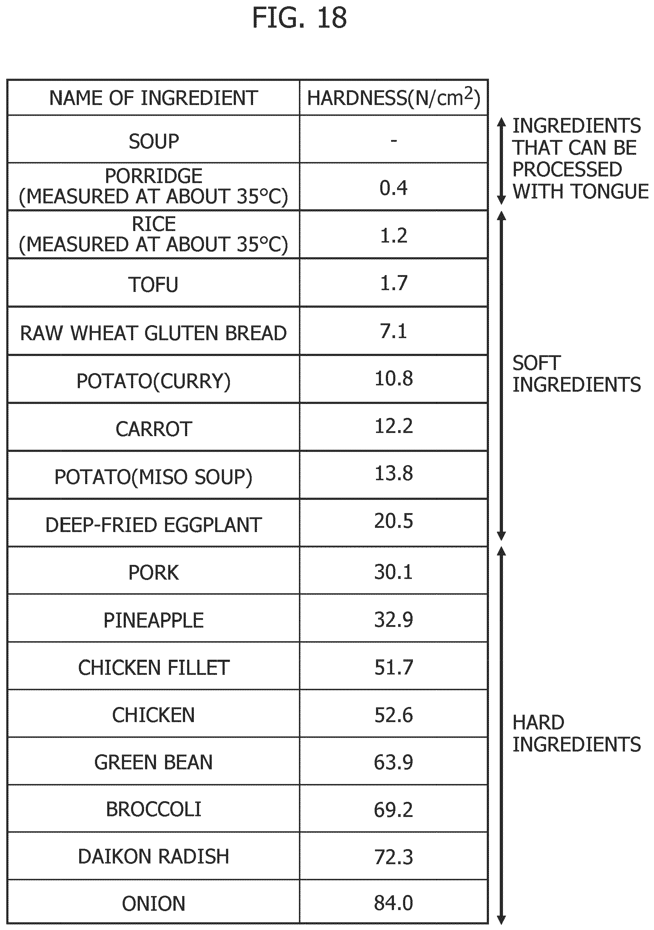

   Fig. 18 is a diagram illustrating an example of ingredients contained in "meal sets".

### DESCRIPTION OF EMBODIMENTS

Hereinafter, embodiments will be described. The embodiments described below are only illustrative examples, and those skilled in the art can appropriately modify the embodiments within an obvious range.

### <Embodiments>

### (Configuration of eating ability evaluation device)

Fig. 1 is a schematic diagram illustrating a configuration of an evaluation device according to the present embodiment. An eating ability evaluation device 1 includes first myoelectric measurement units 11 that are attached to left and right masseter muscle parts of a human subject, a second myoelectric measurement units 12 that is attached to a suprahyoid muscles part of the human subject, and a signal processing unit 14 that is connected to the first myoelectric measurement units 11 and the second myoelectric measurement unit 12.

The first myoelectric measurement units 11 are attached to left and right masseter muscle parts of the human subject and transmit outputs obtained through myoelectric measurement of left and right masseter muscles of the human subject to the signal processing unit 14. Since the masseter muscles are present at two left and right locations, the two first myoelectric measurement units 11 as one set are attached to the right masseter muscle part and the left masseter muscle part of the human subject, respectively. As the first myoelectric measurement units 11 that perform myoelectric measurement of the left and right masseter muscles of the human subject, myoelectric meters for left and right masseter muscles that has already been known can be used.

The second myoelectric measurement unit 12 is attached to the suprahyoid muscles part of the human subject and transmits an outputs obtained through myoelectric measurement of the suprahyoid muscles of the human subject to the signal processing unit 14. As the second myoelectric measurement unit 12 that performs myoelectric measurement of the suprahyoid muscle of the human subject, a myoelectric meter for suprahyoid muscles that has already been known can be used.

The eating ability evaluation device 1 may further include a swallowing measurement unit 13 that is attached to a throat part of the human subject. The swallowing measurement unit 13 is attached to the throat part of the human subject and measures a timing at which food (a drink or a solid product) put into an oral cavity of the human subject is swallowed. As the swallowing measurement unit 13, a microphone, a surface myoelectric meter, or a doppler meter, for example, can be used.

As illustrated in Fig. 1, the signal processing unit 14 includes an electromyogram acquisition unit 21, a left and right masseter muscles work amount acquisition unit 22, a suprahyoid muscles work amount acquisition unit 23, a left and right masseter muscles and suprahyoid muscles work amount ratio acquisition unit 24, a determination unit 25, an evaluation unit 26, and a storage unit 35. The signal processing unit 14 may be realized by hardware or may be realized through cooperation of hardware and software on a computer including a computation unit and a control unit. The signal processing unit 14 receives measurement signals measured by the first myoelectric measurement units 11, the second myoelectric measurement unit 12, and the swallowing measurement unit 13 and performs various kinds of computation. The signal processing unit 14 may be connected to another client or a server via the Internet, a LAN, or a wireless LAN, for example, to exchange information.

The signal processing unit 14 is not limited to a configuration in which all portions configuring the signal processing unit 14 are integrally formed. For example, the electromyogram acquisition unit 21 may be configured separately from the other portions of the signal processing unit 14, and in this case, the first myoelectric measurement units 11, the second myoelectric measurement unit 12, and the electromyogram acquisition unit 21 may be configured by a set of myoelectric data acquisition devices. The left and right masseter muscles work amount acquisition unit 22, the suprahyoid muscles work amount acquisition unit 23, left and right masseter muscles and suprahyoid muscles work amount ratio acquisition unit 24, the determination unit 25, the evaluation unit 26, and the storage unit 35 may be realized through cooperation of hardware and software on one computer.

The electromyogram acquisition unit 21 acquires electromyograms of the left and right masseter muscles from signals of the first myoelectric measurement units 11. Also, the electromyogram acquisition unit 21 acquires an electromyogram of suprahyoid muscles from a signal of the second myoelectric measurement unit 12. The electromyogram of the right masseter muscle and the electromyogram of the left masseter muscles are acquired as the electromyograms of the left and right masseter muscles by the two first myoelectric measurement units 11 being attached as one set to the right masseter muscle part and the left masseter muscle part of the human subject, respectively. Here, the electromyograms are graphs indicating action potentials (µV) of muscles, which are outputs of the first myoelectric measurement units 11 and the second myoelectric measurement unit 12, with respect to an action time (s), and the amount of work (µV·s) of each of the left and right masseter muscles and the suprahyoid muscles is obtained by calculating the areas of the electromyogram waveforms.

The left and right masseter muscles work amount acquisition unit 22 calculates the areas of the waveforms of the electromyograms of the left and right masseter muscles obtained from the signals of the first myoelectric measurement units 11 and acquires the amount of work of the left and right masseter muscles. Here, the amount of work of the left and right masseter muscles is obtained by calculating the area of the waveform of each of the electromyogram of the right masseter muscle and the electromyogram of the left masseter muscle and adding up the areas.

The suprahyoid muscles work amount acquisition unit 23 calculates the area of the waveform of the electromyogram of the suprahyoid muscles obtained from a signal of the second myoelectric measurement unit 12 and acquires the amount of work of the suprahyoid muscles.

In the acquisition of the amounts of work of the left and right masseter muscles and the amounts of work of the suprahyoid muscle, the areas of the waveforms in one masticating and swallowing motion are added up, for example. The one masticating and swallowing motion means a motion until the human subject swallows food to be taken after the human subject puts the food into his/her mouth and performs mastication. The number of times of mastication is, for example, a plurality of times, and may be one or zero.

The left and right masseter muscles and suprahyoid muscles work amount ratio acquisition unit 24 calculates ratios of the amount of work of the left and right masseter muscles obtained by the left and right masseter muscles work amount acquisition unit 22 and the amount of work of the suprahyoid muscles obtained by the suprahyoid muscles work amount acquisition unit 23 and acquires the ratios of the amounts of work of the left and right masseter muscles and suprahyoid muscles. The ratio of the amount of work of the left and right masseter muscles is a ratio of the amount of work of the left and right masseter muscles with respect to the amount of work of oral muscles, which is a total amount of work of the left and right masseter muscles and the suprahyoid muscles. The ratio of the amount of work of the suprahyoid muscles is a ratio of the amount of work of the suprahyoid muscles with respect to the amount of work of the oral muscles. The ratios of the amounts of work of the left and right masseter muscles and the suprahyoid muscles are the ratio of the amount of work of the left and right masseter muscles and the ratio of the amount of work of the suprahyoid muscles. The sum of the ratio of the amount of work of the left and right masseter muscles and the ratio of the amount of work of the suprahyoid muscles is one.

The determination unit 25 determines which of the left and right masseter muscles and the suprahyoid muscles dominantly contribute to mastication on the basis of the ratios of the amounts of work of the left and right masseter muscles and the suprahyoid muscles obtained by the left and right masseter muscles and suprahyoid muscles work amount ratio acquisition unit 24. The determination based on the ratio of the amount of work of the left and right masseter muscles and the ratio of the amount of work of the suprahyoid muscles may be determination based on any one of the ratio of the amount of work of the left and right masseter muscles and the ratio of the amount of work of the suprahyoid muscles.

The evaluation unit 26 evaluates an eating ability on the basis of the ratios of the amounts of work of the left and right masseter muscles and the suprahyoid muscles obtained by the left and right masseter muscles and suprahyoid muscles work amount ratio acquisition unit 24. Evaluation based on the ratio of the amount of work of the left and right masseter muscles and the ratio of the amount of work of the suprahyoid muscles may be evaluation based on any one of the ratio of the amount of work of the left and right masseter muscles and the ratio of the amount of work of the suprahyoid muscles. For example, a representative value of the ratio of the amount of work of the left and right masseter muscles is obtained, and the eating ability is evaluated on the basis of the obtained representative value. Here, the representative value is a mode (mode Mo), a median (median Me), an average value, or the like. It is important to grasp features of motion of the oral muscles during eating for evaluating the eating ability, and a mode indicating the highest detection frequency is preferably adopted. Also, a difference (range R) between a maximum value and a minimum value of the ratio of the amount of work of the left and right masseter muscles may be obtained, and the eating ability may be evaluated on the basis of the obtained range. Moreover, a relationship between the representative value of the ratio of the amount of work of the left and right masseter muscles and the range may be obtained, and the eating ability may be evaluated. Furthermore, the eating ability may be evaluated on the basis of a representative value or a range of the ratio of the amount of work of the suprahyoid muscles, or a relationship between the representative value and the range.

The evaluation unit 26 evaluates the eating ability on the basis of comparison between the ratios of the amounts of work of the left and right masseter muscles and the suprahyoid muscles as targets of evaluation and ratios of the amounts of work of left and right masseter muscles and suprahyoid muscles as targets of comparison. For example, the eating ability is compared on the basis of comparison between a mode of the ratios of the amounts of work of the left and right masseter muscles and the suprahyoid muscles as the targets of evaluation and a mode of ratios of the amounts of work of the left and right masseter muscles and the suprahyoid muscles as the target of comparison. The ratios of the amounts of work of the left and right masseter muscles and the suprahyoid muscles as the targets of comparison are, for example, ratios of the amounts of work of the left and right masseter muscles and the suprahyoid muscles of the same human subject acquired on a different day, ratios of the amounts of work of left and right masseter muscles and suprahyoid muscles of another human subject, or the like. Moreover, a table or the like indicating correlation between the ratios of the amounts of work of the left and right masseter muscles and the suprahyoid muscles and the eating abilities, for example, may be created in advance, and the eating ability may be evaluated with reference to the ratios of the amounts of work of the left and right masseter muscles and the suprahyoid muscles as the targets of evaluation and the table or the like. The table or the like indicating correlation between the ratios of the amounts of work of the left and right masseter muscles and the suprahyoid muscles and the eating ability may be created for each kind of food or can be created as one table by summarizing data on various kinds of food. For example, the level of the eating ability is categorized into two levels, namely a high level and a low level, three levels, namely a high level, a middle level, and a low level, or another multiple levels to perform evaluation. The evaluation based on the ratio of the amount of work of the left and right masseter muscles and the ratio of the amount of work of the suprahyoid muscles may be evaluation based on any one of the ratio of the amount of work of the left and right masseter muscles and the ratio of the amount of work of the suprahyoid muscles.

The signal processing unit 14 may further includes an eating start time measurement unit that measures a time at which food is put into an oral cavity on the basis of a measurement signal received from either the first myoelectric measurement units 11 and the second myoelectric measurement unit 12 and a swallowing time computation unit that obtains a period of time required to swallow the food on the basis of a measurement signal measured by the swallowing measurement unit 13 and the time measured by the eating start time measurement unit.

The signal processing unit 14 can store, in the storage unit 35, each kind of data of signal data of the first myoelectric measurement units 11, signal data of the second myoelectric measurement unit 12, electromyograms acquired by the electromyogram acquisition unit 21, the amount of work of the left and right masseter muscles obtained by the left and right masseter muscles work amount acquisition unit 22, the amount of work of the suprahyoid muscles obtained by the suprahyoid muscles work amount acquisition unit 23, the ratios of the amounts of work of the left and right masseter muscles and the suprahyoid muscles obtained by the ratio acquisition unit 24, the determination result obtained by the determination unit 25, and the evaluation result obtained by the evaluation unit 26 as needed. The data stored in the storage unit 35 can be read from the outside of the evaluation device 1, and it is also possible to use the data for computation performed by the signal processing unit 14 as needed.

Hereinafter, an operation example of the eating ability evaluation device according to the present embodiment will be described. At the time of a start of an operation, each of the first myoelectric measurement units 11, the second myoelectric measurement unit 12, and the swallowing measurement unit 13 is attached to a measurement part of the human subject. Once food is taken by the human subject and a mastication movement of the human subject is started, action of the left and right masseter muscles and the suprahyoid muscles of the human subject becomes active.

Once output signals of the first myoelectric measurement units 11 are input to the signal processing unit 14, electromyograms of the left and right masseter muscles are acquired by the electromyogram acquisition unit 21. Information regarding the electromyograms of the left and right masseter muscles is transmitted to the left and right masseter muscles work amount acquisition unit 22, the areas of the waveforms of the electromyograms of the left and right masseter muscles are calculated, and the amounts of work of the left and right masseter muscles are acquired.

On the other hand, once an output signal of the second myoelectric measurement unit 12 is input to the signal processing unit 14, the electromyogram acquisition unit 21 acquires an electromyogram of the suprahyoid muscles. Information regarding the electromyogram of the suprahyoid muscles is transmitted to the suprahyoid muscles work amount acquisition unit 23, the area of the waveform of the electromyogram of the suprahyoid muscles is calculated, and the amount of work of the suprahyoid muscles is acquired.

The amounts of work of the left and right masseter muscles and the amounts of work of the suprahyoid muscles are transmitted to the left and right masseter muscles and suprahyoid muscles work amount ratio acquisition unit 24, and the ratios of the amounts of work of the left and right masseter muscles and the suprahyoid muscles are acquired. The ratios of the amounts of work of the left and right masseter muscles and the suprahyoid muscles are transmitted to the determination unit 25, which of the left and right masseter muscles and the suprahyoid muscles dominantly contribute to mastication is thereby determined on the basis of the ratios of the amounts of work of the left and right masseter muscles and the suprahyoid muscles, and a determination result is then output. Also, the ratios of the amounts of work of the left and right masseter muscles and the suprahyoid muscles are transmitted to the evaluation unit 26, the eating ability is evaluated on the basis of the ratios of the amounts of work of the left and right masseter muscles and the suprahyoid muscles, and an evaluation result is then output.

The eating start time measurement unit may determine that eating has been started from any one of or both the output signals of the first myoelectric measurement units 11 and the output signal of the second myoelectric measurement unit 12. Also, the swallowing time computation unit may require a period of time required to swallow the food on the basis of the measurement signal measured by the swallowing measurement unit 13 and the time measured by the eating start time measurement unit. The eating start time and the period of time required for swallowing can be used to clarify distinction of kinds of food that are being taken in each example, which will be described later, and can also be used for various kinds of physiological analysis other than the determination of which of the left and right masseter muscles and the suprahyoid muscles dominantly contribute to the mastication and evaluation of the eating ability.

### (Effects and advantages of evaluation device)

In a case of a conventional electromyogram waveform analysis, comparison within a human subject can be performed, while it is difficult to perform comparison among values of the same human subject measured on different days and comparison among human subjects. However, it was found out to be possible to perform the comparison among values of the same human subject measured on different days and the comparison among human subjects by calculating the ratios of the amounts of work of the left and right masseter muscles and the suprahyoid muscles from the amounts of work of the left and right masseter muscles and the suprahyoid muscles.

According to the evaluation device of the present embodiment, it is possible to perform the comparison among values of the same human subject measured on different days and the comparison among human subjects when the amount of work of each of the left and right masseter muscles and the suprahyoid muscles is measured to evaluate an eating ability.

Also, since the mastication is composite movement of moving the lower jaw and the suprahyoid muscles in front-back, up-down, and left-right directions, it is difficult to grasp the mastication movement in detail on the basis only on how large the amounts of work of the left and right masseter muscles and the suprahyoid muscle. According to the evaluation device of the present embodiment, it is possible to grasp the mastication motion, such as mastication motion dominantly performed by the left and right masseter muscles or mastication motion dominantly performed by the suprahyoid muscles, which cannot be evaluated on the basis of how large the amounts of work are.

The evaluation device of the present embodiment can evaluate mastication movement through myoelectric measurement which is non-invasive and simple. The evaluation device is a device as a ground of design of physical properties of products for the purpose of improving maintenance of the mastication ability. It is possible to suggest individual mastication training methods using these products or the evaluation device itself, and it is also possible to observe a change in mastication movement that the training leads to. It is possible to further contribute to an improvement in quality of mastication movement by using the device along with information regarding how large the amounts of work of the left and right masseter muscles and the suprahyoid muscles are.

### (Method of determining muscle that dominantly contributes to eating)

In a method of determining a muscle that dominantly contributes to eating, myoelectric measurement of the left and right masseter muscles during mastication is performed to acquire the amounts of work of the left and right masseter muscles, and also, myoelectric measurement of the suprahyoid muscles is performed to acquire the amounts of work of the suprahyoid muscles first. For example, the electromyogram acquisition unit 21 illustrated in Fig. 1 acquires electromyograms of the left and right masseter muscles from signals of the first myoelectric measurement units 11, and the left and right masseter muscles work amount acquisition unit 22 acquires the amounts of work of the left and right masseter muscles. Also, the electromyogram acquisition unit 21 acquires an electromyogram of the suprahyoid muscles from a signal of the second myoelectric measurement unit 12, and the suprahyoid muscles work amount acquisition unit 23 acquires the amounts of work of the suprahyoid muscles.

Next, the ratios of the amounts of work of the muscles, which are the ratios of the amounts of work of the left and right masseter muscles and the amounts of work of the suprahyoid muscles, are calculated. For example, the left and right masseter muscles and suprahyoid muscles work amount ratio acquisition unit 24 calculates the ratios of the amounts of work of the left and right masseter muscles and the amounts of work of the suprahyoid muscles and acquires the ratios of the amounts of work of the left and right masseter muscles and the suprahyoid muscles.

Subsequently, which of the left and right masseter muscles and the suprahyoid muscles dominantly contribute to mastication is determined on the basis of the obtained ratios of the amounts of work of the muscles. For example, the determination unit 25 determines which of the left and right masseter muscles and the suprahyoid muscles dominantly contribute to mastication on the basis of the ratios of the amounts of work of the left and right masseter muscles and the suprahyoid muscles.

### (Effects and advantages of method of determining muscle that dominantly contribute to eating)

The method of determining a muscle that dominantly contributes to eating in the present embodiment can be used in an evaluation method and an evaluation device capable of performing comparison among values of the same human subject measured on different days and comparison among human subjects when the amount of work of each of left and right masseter muscles and suprahyoid muscles is measured to evaluate the eating ability, and capable of evaluating mastication movement through non-invasive and simple myoelectric measurement. Also, it is possible to grasp mastication motion, such as mastication motion dominantly performed by the left and right masseter muscles or mastication motion dominantly performed by the suprahyoid muscles, which cannot be evaluated on the basis of comparison of how large the amounts of work of the left and right masseter muscles and the suprahyoid muscles are.

### (Method of evaluating eating ability)

In a method of evaluating an eating ability, the amounts of work of the left and right masseter muscles and the amounts of work of the suprahyoid muscles during mastication are acquired, and ratios of the amounts of work of the left and right masseter muscles and the amounts of work of the suprahyoid muscles are calculated, similarly to the method of determining a muscle that dominantly contributes to eating.

Next, which of the left and right masseter muscles and the suprahyoid muscles dominantly contribute to the mastication is determined on the basis of the obtained ratios of the amounts of work of the muscles to evaluate the eating ability. For example, the evaluation unit 26 evaluates the eating ability on the basis of the ratios of the amounts of work of the left and right masseter muscles and the suprahyoid muscles obtained by the left and right masseter muscles and suprahyoid muscles work amount ratio acquisition unit 24.

The evaluation of the eating ability is performed on the basis of the ratios of the amounts of work of the left and right masseter muscles and the suprahyoid muscles obtained by the left and right masseter muscles and suprahyoid muscles work amount ratio acquisition unit 24. The evaluation may be performed on the basis of either the ratios of the amounts of work of the left and right masseter muscles or the ratios of the amounts of work of the suprahyoid muscles. For example, a representative value such as a mode, a median, or an average value of the ratios of the amounts of work of the left and right masseter muscles is obtained, and the eating ability is evaluated on the basis of the obtained representative value. A range of the ratios of the amounts of work of the left and right masseter muscles may be obtained, and the eating ability may be evaluated on the basis of the obtained range. Also, a relationship between the representative value of the ratios of the amounts of work of the left and right masseter muscles and the range may be obtained to evaluate the eating ability.

Also, the evaluation of the eating ability is performed on the basis of comparison between the ratios of the amounts of work of the left and right masseter muscles and the suprahyoid muscles as targets of evaluation and ratios of the amounts of work of left and right masseter muscles and suprahyoid muscles as targets of comparison. The ratios of the amounts of work of the left and right masseter muscles and the suprahyoid muscles as the targets of comparison are, for example, ratios of the amounts of work of the left and right masseter muscles and the suprahyoid muscles of the same human subject acquired on different days, ratios of the amounts of work of left and right masseter muscles and suprahyoid muscles of another human subject, or the like. Moreover, a table or the like indicating correlation between the ratios of the amounts of work of the left and right masseter muscles and the suprahyoid muscles and the eating abilities for each kind of food, for example, may be created in advance, and the eating ability may be evaluated with reference to the ratios of the amounts of work of the left and right masseter muscles and the suprahyoid muscles as the targets of evaluation and the table or the like.

For example, myoelectric measurement of the left and right masseter muscles and myoelectric measurement of the suprahyoid muscles may be performed when a single food is being taken, and which of the left and right masseter muscles and the suprahyoid muscles dominantly contribute to mastication may be determined on the basis of the ratios of the amounts of work of the left and right masseter muscles and the amounts of work of the suprahyoid muscles to evaluate the eating ability. Alternatively, myoelectric measurement of the left and right masseter muscles and myoelectric measurement of the suprahyoid muscles may be performed when a plurality of different kinds of food are being taken, and which of the left and right masseter muscles and the suprahyoid muscles dominantly contribute to mastication may be determined on the basis of the ratios of the amounts of work of the left and right masseter muscles and the amounts of work of the suprahyoid muscles to evaluate the eating ability. The same applies to a case where at least either the representative value or the range of the ratios of the amounts of work of the left and right masseter muscles and the amounts of work of the suprahyoid muscles is calculated and the eating ability is evaluated on the basis of at least any one of them.

### (Effects and advantages of method of evaluating eating ability)

According to the evaluation method of the present embodiment, it is possible to perform comparison among values of the same human subject measured on different days and comparison among human subjects when the amount of work of each of left and right masseter muscles and suprahyoid muscles is measured to evaluate an eating ability. Also, it is possible to grasp mastication motion, such as mastication motion dominantly performed by left and right masseter muscles or mastication motion dominantly performed by suprahyoid muscles, which cannot be evaluated on the basis of how large the amounts of work of the left and right masseter muscles and the suprahyoid muscles are.

According to the evaluation method of the present embodiment, it is possible to evaluate an eating ability by performing myoelectric measurement when a single food or a plurality of different kinds of food is taken and to evaluate differences in mastication motion when the single food or the plurality of different kinds of food is taken. Also, it is possible to monitor mastication motion when food is successively taken in a meal and to evaluate the eating ability. Moreover, it is possible to evaluate the eating ability by reflecting differences in a plurality of mastication motions and a mastication ability in a meal, which cannot be grasped on the basis of how large the amount of work when a single food is taken is.

### <Example 1>

The first myoelectric measurement units 11 and the second myoelectric measurement unit 12 were attached to each of ten human subjects by using the evaluation device 1 illustrated in Fig. 1, myoelectric measurement of left and right masseter muscles and suprahyoid muscles when the same food was taken was performed, and electromyograms were acquired. In this example, the food was a curry rice set (a salad including green beans and ham, curry rice including potatoes, fukujinzuke pickles, and peaches as a dessert).

Fig. 2 is a schematic diagram for explaining a state where the myoelectric measurement units for left and right masseter muscles and suprahyoid muscles of the evaluation device 1 in Fig. 1 were attached to each human subject. The two first myoelectric measurement units 11 as a set were attached to left and right masseter muscle parts of each human subject, and the second myoelectric measurement unit 12 was attached to suprahyoid muscles part of each human subject.

Fig. 3 is an example of (A) an electromyogram of a right masseter muscle, (B) an electromyogram of a left masseter muscle, and (C) an electromyogram of suprahyoid muscles acquired in this example. The vertical axis of each electromyogram represents a myoelectric meter output signal (unit: µV), and the horizontal axis represents a time (unit: s). Each electromyogram was acquired by the electromyogram acquisition unit 21 from signals of the first myoelectric measurement units 11 and a signal of the second myoelectric measurement unit 12. In Fig. 3 (A) to (C), the signal of each electromyogram is divided for each kind of taken food and indicates a signal fa when curry rice was taken, a signal fb when fukujinzuke pickles was taken, and a signal fc when green beans were taken.

The areas of waveforms of the electromyograms of the right masseter muscle and the left masseter muscle were calculated from the data of the electromyograms in Fig. 3 (A) and (B), the areas of the waveforms in one masticating and swallowing motion were added up, and the amount of work of the left and right masseter muscles in one masticating and swallowing motion was acquired. Also, the area of a waveform of the electromyogram of the suprahyoid muscles was calculated from data of the electromyogram in Fig. 3 (C), and the amount of work of the suprahyoid muscles in one masticating and swallowing motion was acquired.

A plurality of specialists including dentists conducted tests at the same time in regard to evaluation items related to eating abilities from states of eating of the ten human subjects when they ate the food to acquire the electromyograms. The eating abilities of the ten human subjects were categorized into three levels, namely a high level, a middle level, and a low level, and determined on the basis of total scores of the items. Details of the evaluation items, scores of the human subjects, and determination of the eating abilities are summarized and shown in Table 1.

**[Table 1]**

| HUMAN SUBJECT NO. | 6 | 1 | 9 | 7 | 11 | 8 | 2 | 10 | 4 | 5 |
|---|---|---|---|---|---|---|---|---|---|---|
| HAVE A DIFFICULTY IN EATING HARD STUFF (LUMP OF MEAT) | × | × | × | × | × | × | ○ | ● | ● | ○ |
| HAVE A DIFFICULTY IN EATING HARD STUFF (SLICED THIN MEAT) | × | × | × | × | × | × | × | ● | ○ | ● |
| HAVE A DIFFICULTY IN EATING HARD STUFF (FUKUJINZUKE PICKLES) | × | × | × | × | × | × | × | × | ● | ● |
| HAVE A DIFFICULTY IN EATING A FIBROUS INGREDIENT (GREEN VEGETABLE STEM PARTS) | × | × | × | × | × | × | × | ● | ● | × |
| HAVE A DIFFICULTY IN EATING A FIBROUS INGREDIENT (PINEAPPLES) | × | × | × | × | × | × | × | × | × | ● |
| HAVE A DIFFICULTY IN EATING A FIBROUS INGREDIENT (CITRUS PIECES) | × | × | × | - | ● | ● | ● | - | × | ● |
| EASILY CHOKE ON TEA AND SOUP | × | × | × | ● | × | × | ● | ● | ● | × |
| USE ARTIFICIAL TEETH | × | ● | ● | ● | ● | ● | ● | ● | ● | ● |
| SCORE | 0 | 2 | 2 | 4 | 4 | 4 | 7 | 8 | 9 | 11 |
| EATING ABILITY | HIGH | | | MIDDLE | | | | LOW | | |

| | | | | | | | | | | |
|---|---|---|---|---|---|---|---|---|---|---|
| • :APPLIED (2 POINTS) ∘:SLIGHTLY APPLIED (1 POINT) ×:NOT APPLIED (0 POINTS) | | | | | | | | | | |

The evaluation items related to the eating abilities are items such as "have a difficulty in eating hard stuff (lump of meat)" and "have a difficulty in hard stuff (sliced thin meat)" as shown in Table 1. The evaluation items are not limited thereto, and there may be items with content other than those shown in Table 1. For each evaluation item, 2 points were applied when a human subject met the evaluation item, 1 point was applied when the human subject slightly met the evaluation item, and 0 points were applied when the human subject did not meet the evaluation item. Human subjects with total scores of 0 to 3 points when the scores of items (human subject Nos. 6, 1, and 9) were added up were categorized into a group of a high eating ability, human subjects with total scores of 4 to 7 points (human subject Nos. 7, 11, 8, and 2) were categorized into a group of a middle eating ability, and human subjects with total scores of 8 to 11 points (human subject Nos. 10, 4, and 5) were categorized into a group of a low eating ability.

Fig. 4A is an example of a graph indicating the amounts of work (µV·s) of left and right masseter muscles and suprahyoid muscles in one masticating and swallowing motion of a human subject. The graph of each amount of work is illustrated in a divided manner for each kind of the taken food to correspond to the signal fa when the curry rice was taken, the signal fb when the fukujinzuke pickles were taken, and the signal fc when the green beans were taken.

Fig. 4B is an example of a graph indicating ratios of the amounts of work of left and right masseter muscles and suprahyoid muscles in one masticating and swallowing motion of a human subject. The ratios of the amounts of work of the left and right masseter muscles and the suprahyoid muscles were obtained from the data of the amounts of work of the left and right masseter muscles and the amount of work of the suprahyoid muscles in Fig. 4A.

Fig. 5A is a graph indicating ratios (%) of the amounts of work of the left and right masseter muscles and the suprahyoid muscles in one masticating and swallowing motion of the human subjects categorized into the group of the high eating ability in the result of the tests of the evaluation items related to the eating abilities described above. The graph of the ratio of each amount of work is illustrated for each kind of taken food so as to correspond to the signal fa when the curry rice set was taken, the signal fb when the fukujinzuke pickles were taken, and the signal fc when the green beans were taken.

The ratios of the amounts of work of the left and right masseter muscles and the suprahyoid muscles when the human subjects categorized into the group of the eating abilities in the middle level and the human subjects categorized into the group of the eating abilities in the low level as a result of the tests of the evaluation items related to the eating abilities took the curry rice set were also acquired.

Fig. 5B is a graph indicating ratios of the amounts of work of the left and right masseter muscles and the suprahyoid muscles in one masticating and swallowing motion of the human subjects with middle eating abilities acquired in this example. Fig. 5C is a graph indicating ratios of the amounts of work of the left and right masseter muscles and the suprahyoid muscles in one masticating and swallowing motion of the human subjects with low eating abilities acquired in this example. The graph of the ratio of each amount of work is illustrated for each kind of taken food so as to correspond to the signal fa when the curry rice set was taken, the signal fb when the fukujinzuke pickles were taken, and the signal fc when the green beans were taken.

It was confirmed from the comparison of Figs. 5A to 5C that the human subjects with the ratios of the amounts of work of the left and right masseter muscles that were as high as about 70% to 80% had high eating abilities, the human subjects with the ratios of the amounts of work of the left and right masseter muscles that were as low as about 40% to 60% had low eating abilities, and the human subjects with the ratios of the amounts of work of the left and right masseter muscles in a middle level had middle eating abilities, in eating of the curry rice set.

Fig. 6A is a graph indicating, for each kind of taken food, the ratios of the amounts of work of the left and right masseter muscles in one masticating and swallowing motion of the human subjects with the high eating abilities acquired in this example. Fig. 6A illustrates the ratios of the amounts of work of the left and right masseter muscles and the suprahyoid muscles in one masticating and swallowing motion of the food indicated along the horizontal axis. In a case where motions are indicated instead of food along the horizontal axis, ratios of the amounts of work of the left and right masseter muscles and the suprahyoid muscles during the motions are indicated.

Fig. 6B is a graph indicating the ratios of the amounts of work of the left and right masseter muscles in one masticating and swallowing motion of the human subjects with the high eating abilities acquired in Example 1. A mode Mo, a median Me, and a range R of the ratios of the amounts of work of the left and right masseter muscles as illustrated in Fig. 6B were obtained, and evaluation of the eating abilities was conducted.

Fig. 6C is a diagram in which the horizontal axis represents a mode or a median of the ratios of the amounts of work of the left and right masseter muscles and the vertical axis represents a range. It is possible to make the following determination in relation to the evaluation of the eating abilities from the graph obtained by indicating the mode Mo or the median Me and the range R of the ratios of the amounts of work of the left and right masseter muscles obtained in the above on the diagram illustrated in Fig. 6C. For example, it is possible to determine that the left and right masseter muscles dominantly worked and the number of movement varieties was large in a case where the mode Mo or the median Me of the ratios of the left and right masseter muscles was high and the range R was large (the first quadrant in Fig. 6C). It is possible to determine that the suprahyoid muscles dominantly worked and the number of movement varieties was large in a case where the mode Mo or the median Me of the ratios of the left and right masseter muscles was low and the range R was large (the second quadrant in Fig. 6C). It is possible to determine that the suprahyoid muscles dominantly worked and the number of movement varieties was small in a case where the mode Mo or the median Me of the ratios of the left and right masseter muscles was low and the range R was small (the third quadrant in Fig. 6C). It is possible to determine that the left and right masseter muscles dominantly worked and the number of movement varieties was small in a case where the mode Mo or the median Me of the ratios of the left and right masseter muscles was high and the range R was small (the fourth quadrant in Fig. 6C).

### <Example 2>

Eating tests of acquiring electromyograms when human subjects with high, middle, and low eating abilities ate curry rice sets and acquiring, for each kind of taken food, ratios of the amounts of work of left and right masseter muscles and suprahyoid muscle were conducted using the evaluation device 1 illustrated in Fig. 1.

Fig. 7A is a graph indicating, for each kind of taken food, ratios of the amounts of work of the left and right masseter muscles and the suprahyoid muscles in one masticating and swallowing motion of the human subject with the high eating ability acquired in this example. Also, Fig. 7B is a graph indicating, for each kind of taken food, ratios of the amounts of work of the left and right masseter muscles and the suprahyoid muscles in one masticating and swallowing motion of the human subject with the middle eating ability acquired in this example, and Fig. 7C is a graph indicating, for each kind of taken food, ratios of the amounts of work of the left and right masseter muscles and the suprahyoid muscles in one masticating and swallowing motion of the human subject with the low eating ability acquired in this example. Each graph indicates the ratios of the amounts of work of the left and right masseter muscles and the suprahyoid muscles in one masticating and swallowing motion of the food indicated along the horizontal axis. In a case where motions are indicated along the horizontal axis instead of food, the ratios of the amounts of work of the left and right masseter muscles and the suprahyoid muscles during the motions are indicated.

In comparison of the three graphs in Figs. 7A to 7C, the ratios of the amounts of work of the left and right masseter muscles were generally higher than the ratios of the amounts of work of the suprahyoid muscles regardless of the kinds of food and the motions in the human subject with the high eating ability, while the ratios of the amounts of work of the left and right masseter muscles and the ratios of the amounts of work of the suprahyoid muscles were similar (the left and right masseter muscles and the suprahyoid muscles were similar) in the human subject with the low eating ability, although the kinds of taken food and motions were not common. Also, a result in a middle level of the above results was obtained for the human subject with the middle eating ability.

Fig. 7D is a graph indicating ratios of the amounts of work of left and right masseter muscles in one masticating and swallowing motion of each of human subjects with high, middle, and low eating abilities acquired in this example. The mode Mo, the median Me, and the range R of the ratios of the amounts of work of the left and right masseter muscles of the human subject with the high eating ability were 66%, 64%, and 39.55, respectively. The mode Mo, the median Me, and the range R of the ratios of the amounts of work of the left and right masseter muscles of the human subject with the middle eating ability were 56%, 57%, and 36.36, respectively. The mode Mo, the median Me, and the range R of the amounts of work of the left and right masseter muscles of the human subject with the low eating ability were 56%, 52%, and 19.37, respectively.

Fig. 7E is a graph indicating a range with respect to the median of the ratios of the amounts of work of the left and right masseter muscles in this example. Fig. 7F is a graph indicating a range with respect to a mode of the ratios of the amounts of work of the left and right masseter muscles in this example. Figs. 7E and 7F indicate that in general, the median Me, the mode Mo, and the range R of the amounts of work of the left and right masseter muscles were larger, the left and right masseter muscles relatively more dominantly worked, and the number of movement varieties was larger as the eating ability was higher. The results show that the median Me, the mode Mo, and the range R of the amounts of work of the left and right masseter muscles were smaller, the left and right masseter muscles relatively less dominantly worked, and the number of movement varieties was smaller as the eating ability was lower.

As described above, it was possible to perform comparison among human subjects when the eating abilities were evaluated, by obtaining and comparing the ratios of the amounts of work of the left and right masseter muscles and the suprahyoid muscles. It was also possible to perform comparison among values of the same human subject measured on different days. Also, it was possible to grasp the mastication motion, such as mastication motion dominantly performed by the left and right masseter muscles or mastication motion dominantly performed by the suprahyoid muscles, which was not able to be evaluated on the basis of how large the amounts of work of the left and right masseter muscles and the suprahyoid muscle were.

In the above evaluation of eating abilities, a table or the like indicating correlation between the ratios of the amounts of work of the left and right masseter muscles and the suprahyoid muscles and eating abilities for each kind of food, for example, may be created in advance, and the eating abilities may be evaluated with reference to the ratios of the amounts of work of the left and right masseter muscles and the suprahyoid muscles obtained through the measurement of the human subjects and the above table or the like. For example, the above table is a table indicating correlation in which the ratios of the amounts of work of the left and right masseter muscles were generally higher than the ratios of the amounts of work of the suprahyoid muscles regardless of the kinds of food and motions in the human subject with the high eating ability (the left and right masseter muscles dominantly worked) while the ratios of the amounts of work of the left and right masseter muscles and the ratios of the amounts of work of the suprahyoid muscles were similar (the left and right masseter muscles and the suprahyoid muscles were similar) in the human subject with the low eating ability in eating of curry rice sets. Once the table is created, it is possible to evaluate the eating abilities on the basis of the amounts of work of the left and right masseter muscles and the suprahyoid muscles with reference to the tables if the kinds of the food are similar.

### <Example 3>

Similarly to Example 2, eating tests of acquiring electromyograms when human subjects with high, middle, and low eating abilities ate somen noodle sets and acquiring ratios of the amounts of work of left and right masseter muscles and suprahyoid muscles for each kind of taken food were conducted. Each somen noodle set was a set including somen noodle containing fillet meat, dressed rapeseed blossoms, fried daikon radish, and pineapples as dessert.

Fig. 8A is a graph indicating, for each kind of taken food, ratios of the amounts of work of left and right masseter muscles and suprahyoid muscles in one masticating and swallowing motion of the human subject with the high eating ability acquired in this example. Also, Fig. 8B is a graph indicating, for each kind of taken food, ratios of the amounts of work of left and right masseter muscles and suprahyoid muscles in one masticating and swallowing motion of the human subject with the middle eating ability acquired in this example, and Fig. 8C is a graph indicating, for each kind of taken food, ratios of the amounts of work of left and right masseter muscles and suprahyoid muscles in one masticating and swallowing motion of the human subject with the low eating ability acquired in this example. Each graph indicates the ratios of the amounts of work of the left and right masseter muscles and the suprahyoid muscles in one masticating and swallowing motion of food indicated along the horizontal axis. In a case where motions are indicated along the horizontal axis instead of food, ratios of the amounts of work of the left and right masseter muscles and the suprahyoid muscles during the motions are indicated.

In comparison of the three graphs in Figs. 8A to 8C, the ratios of the amounts of work of the left and right masseter muscles were generally higher than the ratios of the amounts of work of the suprahyoid muscles (the left and right masseter muscles dominantly worked) regardless of the kinds of food and the motions in the human subject with the high eating ability, while the ratios of the amounts of work of the left and right masseter muscles and the ratios of the amounts of work of the suprahyoid muscles were similar (the left and right masseter muscles and the suprahyoid muscles were similar) in the human subject with the low eating ability, although the kinds of taken food and the motions were not common. Also, a result in a middle level of the above results was obtained for the human subject with the middle eating ability.

Fig. 8D is a graph indicating the ratios of the amounts of work of the left and right masseter muscles in one masticating and swallowing motion of each of the human subjects with the high, middle, and low eating abilities acquired in this example. The mode Mo, the median Me, and the range R of the ratios of the amounts of work of the left and right masseter muscles of the human subject with the high eating ability were 60%, 60%, and 33.19, respectively. The mode Mo, the median Me, and the range R of the ratios of the amounts of work of the left and right masseter muscles of the human subject with the middle eating ability were 60%, 52%, and 34.15, respectively. The mode Mo, the median Me, and the range R of the ratios of the amounts of work of the left and right masseter muscles of the human subject with the low eating ability were 50%, 50%, and 15.41, respectively.

Fig. 8E is a graph indicating a range with respect to a median of the ratios of the amounts of work of the left and right masseter muscles in this example. Fig. 8F is a graph indicating a range with respect to a mode of the ratios of the amounts of work of the left and right masseter muscles in this example. Figs. 8E and 8F indicate that in general, the median Me, the mode Mo, and the range R of the amounts of work of the left and right masseter muscles were larger, the left and right masseter muscles relatively more dominantly worked, and the number of movement varieties was larger as the eating ability was higher. The results show that the median Me, the mode Mo, and the range R of the amounts of work of the left and right masseter muscles were smaller, the left and right masseter muscles relatively less dominantly worked, and the number of movement varieties was smaller as the eating ability was lower.

As described above, it was possible to perform comparison among human subjects when eating abilities were evaluated, by obtaining and comparing the ratios of the amounts of work of the left and right masseter muscles and the suprahyoid muscles. It was also possible to perform comparison among values of the same human subject measured on different days. Also, it was possible to grasp the mastication motion, such as mastication motion dominantly performed by the left and right masseter muscles and mastication motion dominantly performed by the suprahyoid muscles, which was not able to be evaluated on the basis of how large the amounts of work of the left and right masseter muscles and the suprahyoid muscle were.

### <Example 4>

Similarly to Example 2, eating tests of acquiring electromyograms when human subjects with high, middle, and low eating abilities ate fried pork and dressed broccoli sets and acquiring ratios of the amounts of work of left and right masseter muscles and suprahyoid muscles for each kind of taken food were conducted. Each fried pork and dressed broccoli set is a set including fried pork containing green peas, onions, pork, and the like, rice, dressed broccoli, pickles, miso soup, and navel oranges as dessert.

Fig. 9A is a graph indicating, for each kind of taken food, ratios of the amounts of work of left and right masseter muscles and the suprahyoid muscles in one masticating and swallowing motion of the human subject with the high eating ability acquired in this example. Also, Fig. 9B is a graph indicating, for each kind of taken food, ratios of the amounts of work of left and right masseter muscles and suprahyoid muscles in one masticating and swallowing motion of the human subject with the middle eating ability acquired in this example, and Fig. 9C is a graph indicating, for each kind of taken food, ratios of the amounts of work of left and right masseter muscles and suprahyoid muscles in one masticating and swallowing motion of the human subject with the low eating ability acquired in this example. Each graph indicates ratios of the amounts of work of the left and right masseter muscles and the suprahyoid muscles in one masticating and swallowing motion of the food indicated along the horizontal axis. In a case where motions are indicated along the horizontal axis instead of food, the ratios of the amounts of work of the left and right masseter muscles and the suprahyoid muscles during the motions are indicated. Rice was replaced with porridge in eating of the human subject with the middle eating ability illustrated in Fig. 9B.

In comparison of the three graphs in Figs. 9A to 9C, the ratios of the amounts of work of the left and right masseter muscles were generally higher than the ratios of the amounts of work of the suprahyoid muscles (the left and right masseter muscles dominantly worked) regardless of the kinds of food and the motions in the human subject with the high eating ability, while the ratios of the amounts of work of the left and right masseter muscles and the ratios of the amounts of work of the suprahyoid muscles were similar (the left and right masseter muscles and the suprahyoid muscles were similar) in the human subject with the low eating ability, although the kinds of taken food and the motions were not common. Also, a result in a middle level of the above results was obtained for the human subject with the middle eating ability.

Fig. 9D is a graph indicating ratios of the amounts of work of the left and right masseter muscles in one masticating and swallowing motion of each of human subjects with high, middle, and low eating abilities acquired in this example. The mode Mo, the median Me, and the range R of the ratios of the amounts of work of the left and right masseter muscles of the human subject with the high eating ability were 78%, 69%, and 51.73, respectively. The mode Mo, the median Me, and the range R of the ratios of the amounts of work of the left and right masseter muscles of the human subject with the middle eating ability were 50%, 54%, and 35.82, respectively. The mode Mo, the median Me, and the range R of the ratios of the amounts of work of the left and right masseter muscles of the human subject with the low eating ability were 44%, 47%, and 43.51.

Fig. 9E is a graph indicating a range with respect to a median of the ratios of the amounts of work of the left and right masseter muscles in this example. Fig. 9F is a graph indicating a range with respect to a mode of the ratios of the amounts of work of the left and right masseter muscles in this example. Figs. 9E and 9F indicate that in general, the median Me, the mode Mo, and the range R of the amounts of work of the left and right masseter muscles were large, the left and right masseter muscles relatively dominantly worked, and the number of movement varieties was large in the human subject with the high eating ability. Although some part of the order was reversed for the human subject with the middle eating ability since the rice was replaced with porridge, the results show that the medians Me, the modes Mo, and the ranges R of the amounts of work of the left and right masseter muscles were small, the left and right masseter muscles relatively less dominantly worked, and the numbers of movement varieties were small in the human subjects with the low and middle eating abilities.

As described above, it was possible to perform comparison among human subjects when eating abilities were evaluated, by obtaining and comparing the ratios of the amounts of work of the left and right masseter muscles and the suprahyoid muscles. It was also possible to perform comparison among values of the same human subject measured on different days. Also, it was possible to grasp the mastication motion, such as mastication motion dominantly performed by the left and right masseter muscles and mastication motion dominantly performed by the suprahyoid muscles, which was not able to be evaluated on the basis of how large the amounts of work of the left and right masseter muscles and the suprahyoid muscle were.

### <Example 5>

Similarly to Example 2, eating tests of acquiring electromyograms when human subjects with high, middle, and low eating abilities ate fried pork and hiyayakko tofu sets and acquiring ratios of the amounts of work of left and right masseter muscles and suprahyoid muscles for each kind of taken food. Each fried pork and hiyayakko tofu set is a set including fried pork including cabbages, carrots, eggplants, green peppers, meat, and the like, hiyayakko tofu (tofu), and clear soup (clams).

Fig. 10A is a graph indicating, for each kind of taken food, ratios of the amounts of work of left and right masseter muscles and suprahyoid muscles in one masticating and swallowing motion of a human subject with a high eating ability acquired in this example. Also, Fig. 10B is a graph indicating, for each kind of taken food, ratios of the amounts of work of left and right masseter muscles and suprahyoid muscles in one masticating and swallowing motion of a human subject with a middle eating ability acquired in this example, and Fig. 10C is a graph indicating, for each kind of taken food, ratios of the amounts of work of left and right masseter muscles and suprahyoid muscles in one masticating and swallowing motion of a human subject with a low eating ability acquired in this example. Each graph indicates the ratios of the amounts of work of the left and right masseter muscles and the suprahyoid muscles in one masticating and swallowing motion of the food indicated along the horizontal axis. In a case where motions are indicated along the horizontal axis instead of food, the ratios of the amounts of work of the left and right masseter muscles and the suprahyoid muscles during the motions are indicated. In eating of the human subject with the middle eating ability illustrated in Fig. 10B, rice was replaced with porridge.

In comparison of the three graphs in Figs. 10A to 10C, the ratios of the amounts of work of the left and right masseter muscles were generally higher than the ratios of the amounts of work of the suprahyoid muscles regardless of the kinds of food and the motions (the left and right masseter muscles dominantly worked) in the human subject with the high eating ability, while the ratios of the amounts of work of the suprahyoid muscles were higher than the ratios of the amounts of work of the left and right masseter muscles (the suprahyoid muscles dominantly worked) in the human subject with the low eating ability, although the kinds of taken food and the motions were not common. Also, a result in a middle level of the above results was obtained for the human subject with the middle eating ability.

Fig. 10D is a graph indicating ratios of the amounts of work of the left and right masseter muscles in one masticating and swallowing motion of each of the human subjects with the high, middle, and low eating abilities acquired in this example. The mode Mo, the median Me, and the range R of the ratios of the amounts of work of the left and right masseter muscles in the human subject with the high eating ability were 52%, 55%, and 57.02, respectively. The mode Mo, the median Me, and the range R of the ratios of the amounts of work of the left and right masseter muscles of the human subject with the middle eating ability were 53%, 44%, and 28.33, respectively. The mode Mo, the median Me, and the range R of the ratios of the amounts of work of the left and right masseter muscles of the human subject with the low eating ability were 38%, 35%, and 24.93.

Fig. 10E is a graph indicating a range with respect to a median of the ratios of the amounts of work of the left and right masseter muscles in this example. Fig. 10F is a graph indicating a range with respect to a mode of the ratios of the amounts of work of the left and right masseter muscles in this example. Figs. 10E and 10F indicate that in general, the median Me, the mode Mo, and the range R of the amounts of work of the left and right masseter muscles were large, the left and right masseter muscles relatively dominantly worked, and the number of movement varieties was large in the human subject with the eating ability. The results show that the medians Me, the modes Mo, and the ranges R of the amounts of work of the left and right masseter muscles were small, the left and right masseter muscles relatively less dominantly worked, and the numbers of movement varieties were small in the human subjects with the low and middle eating abilities.

As described above, it was possible to perform comparison among human subjects when eating abilities were evaluated, by obtaining and comparing the ratios of the amounts of work of the left and right masseter muscles and the suprahyoid muscles. It was also possible to perform comparison among values of the same human subject measured on different days. Also, it was possible to grasp the mastication motion, such as mastication motion dominantly performed by the left and right masseter muscles or mastication motion dominantly performed by the suprahyoid muscles, which was not able to be evaluated on the basis of how large the amounts of work of the left and right masseter muscles and the suprahyoid muscle were.

### <Example 6>

Fig. 11A is a graph indicating a median Me of ratios of the amounts of work of left and right masseter muscles with respect to a score of tests of evaluation items related to an eating ability in this example. Data of the median Me of the ratios of the amounts of work of the left and right masseter muscles in the graph illustrated in Fig. 11A is data obtained through measurement in total of five eating tests on the human subjects with the high eating abilities, data obtained through measurement in total of five eating tests on the human subjects with the middle eating abilities, and data obtained through measurement in total of five eating tests on the human subjects with the low eating abilities, including the data in Examples 2 to 5 above. There was a trend that the median Me of the ratios of the amounts of work of the left and right masseter muscles decreased as the score of the evaluation items related to the eating ability increases. In the drawing, a straight line y = -1.4407x + 62.428 obtained through the least square method is shown. Here, x is a score of the evaluation items related to the eating ability, and y is a median Me of the ratio of the amounts of work of the left and right masseter muscles. When the human subjects of not more than 3 points as a total score of the test of the evaluation items related to the eating ability are categorized into a group of a high eating ability, the human subjects of not less than 8 points are categorized into a group of a low eating ability, and the human subjects in a region therebetween are categorized as a group of a middle eating ability, it is possible to categorize the human subjects with the medians Me of the ratios of the amounts of work of the left and right masseter muscles that are not less than 58.1% into the group of the high eating ability, to categorize the human subjects with the medians Me of the ratios of the amounts of work of the left and right masseter muscles that are not more than 50.9% into the group of the low eating ability, and to categorize the human subjects with the medians Me of the ratios of the amounts of work of the left and right masseter muscles that are more than 50.9% and less than 58.1% into the group of the middle eating ability. It is possible to create a table indicating correlation between the medians Me of the ratios of the amounts of work of the left and right masseter muscles and the suprahyoid muscles and the eating ability from the data in Fig. 11A in advance. It is possible to evaluate the eating ability with reference to the above table when the ratios of the amounts of work of the left and right masseter muscles and the suprahyoid muscles of the target of evaluation are acquired.

Fig. 11B is a graph indicating a mode Mo of the ratios of the amounts of work of the left and right masseter muscles with respect to a score of the tests of the evaluation items related to the eating ability in this example. Data of the mode Mo of the ratios of the amounts of work of the left and right masseter muscles in the graph illustrated in Fig. 11B is data measured similarly to the data illustrated in Fig. 11A. There is a trend that the mode Mo of the ratios of the amounts of work of the left and right masseter muscles decreases as the score of the evaluation items related to the eating ability increases. In the drawing, a straight line y = -1.6547x + 65.369 obtained by the least square method is illustrated. Here, x is a score of the evaluation items related to the eating ability, and y is a mode Mo of the ratios of the amounts of work of the left and right masseter muscles. When the human subjects of not more than 3 points as a total score of the test of the evaluation items related to the eating ability are categorized into a group of a high eating ability, the human subjects of not less than 8 points are categorized into a group of a low eating group, and the human subjects in a region therebetween are categorized into a group of a middle eating ability, it is possible to categorize the human subjects with the modes Mo of the ratios of the amounts of work of the left and right masseter muscles that are not less than 60.4% into the group of the high eating ability, to categorize the human subjects with the modes Mo of the ratios of the amounts of work of the left and right masseter muscles that are not more than 52.1% into the group of the low eating ability, and to categorize the human subjects with the modes Mo of the ratios of the amounts of work of the left and right masseter muscles that are more than 52.1% and less than 60.4% into the group of the middle eating ability. It is possible to create a table indicating correlation between the mode Mo of the ratios of the amounts of work of the left and right masseter muscles and the suprahyoid muscles and the eating ability from the data in Fig. 11B in advance. It is possible to evaluate the eating ability with reference to the above table when the ratios of the amounts of work of the left and right masseter muscles and the suprahyoid muscles of the target of evaluation are acquired.

Fig. 11C is a graph indicating a range R of the ratios of the amounts of work of left and right masseter muscles with respect to a score of the tests of the evaluation items related to the eating ability in this example. Data of the range R of the ratios of the amounts of work of the left and right masseter muscles in the graph illustrated in Fig. 11C is data measured similarly to the data illustrated in Fig. 11A. There is a trend that the range R of the ratios of the amounts of work of the left and right masseter muscles decreases as the score of the evaluation items related to the eating ability increases. In the drawing, a straight line y = -1.9781x + 47.659 obtained by the least square method is illustrated. Here, x is a score of the evaluation items related to the eating ability, and y is a range R of the ratios of the amounts of work of the left and right masseter muscles. When the human subjects of not more than 3 points as a total score of the tests of the evaluation items related to the eating ability are categorized into a group of a high eating ability, the human subjects of not less than 8 points are categorized into a group of a low eating ability, and the human subjects in the region therebetween are categorized into a group of a middle eating ability, it is possible to categorize the human subjects with the ranges R of the ratios of the amounts of work of the left and right masseter muscles that are not less than 41.7 into the group of the high eating ability, to categorize the human subjects with the ranges R of the ratios of the amounts of work of the left and right masseter muscles that are not more than 31.8 into the group of the low eating ability, and to categorize the human subjects with the ranges R of the ratios of the amounts of work of the left and right masseter muscles that are more than 31.8 and less than 41.7 into the group of the middle eating ability. It is possible to create a table indicating correlation between the range R of the ratios of the amounts of work of the left and right masseter muscles and the suprahyoid muscles and the eating ability from the data in Fig. 11C in advance. It is possible to evaluate the eating ability with reference to the above table when the ratios of the amounts of work of the left and right masseter muscles and the suprahyoid muscles of the target of evaluation are acquired.

Fig. 11D is a graph indicating the range R with respect to the median Me of the ratios of the amounts of work of the left and right masseter muscles in this example. Data of the median Me and the range R of the ratios of the amounts of work of the left and right masseter muscles in the graph illustrated in Fig. 11D is the same as the data illustrated in Figs. 11A and 11C. In Fig. 11D, thick lines are illustrated at locations where the median Me of the ratios of the amounts of work of the left and right masseter muscles are 58.1% and 50.9%, which are defined as boundaries to distinguish the high, middle, and low eating abilities from Fig. 11A. Also, thick lines are illustrated at locations where the range R of the ratios of the amounts of work of the left and right masseter muscles are 41.7 and 31.8, which are defined as boundaries to distinguish the high, middle, and low eating ability from Fig. 11C.

Fig. 11E is a graph indicating the range R with respect to the mode Mo of the ratios of the amounts of work of the left and right masseter muscles in this example. Data of the mode Mo and the range R of the ratios of the amounts of work of the left and right masseter muscles in the graph illustrated in Fig. 11E is the same as the data illustrated in Figs. 11B and 11C. In Fig. 11E, thick lines are indicated at locations where the mode Mo of the ratios of the amounts of work of the left and right masseter muscles is 60.4% and 52.1%, which are defined as boundaries to distinguish the high, middle, and low eating abilities from Fig. 11B. Also, thick lines are illustrated at locations where the range R of the ratios of the amounts of work of the left and right masseter muscles are 41.7 and 31.8, which are defined as boundaries to distinguish the high, middle, and low eating abilities from Fig. 11C.

As illustrated in Figs. 11D and 11E, it was confirmed that in general, the median Me, the mode Mo, and the range R of the amounts of work of the left and right masseter muscles were larger, the left and right masseter muscles relatively more dominantly worked, and the number of movement varieties was larger as the eating ability was higher. Also, it was confirmed that the median Me, the mode Mo, and the range R of the amounts of work of the left and right masseter muscles were smaller, the left and right masseter muscles relatively less dominantly worked, and the number of movement varieties was smaller as the eating ability was lower. This is considered to correspond to the fact that the mode Mo or the median Me of the ratios of the left and right masseter muscles is higher, the range R is larger, the data is present closer to the first quadrant, the left and right masseter muscles more dominantly work, and the number of movement varieties is larger in Fig. 6C as the eating ability was higher, while the mode Mo or the median Me of the ratios of the left and right masseter muscles is lower, the range R is smaller, the data is present closer to the third quadrant, the left and right masseter muscles less dominantly work, and the number of movement varieties is smaller in Fig. 6C as the eating ability decreases.

Fig. 12 is a diagram for explaining ratios of the amounts of work of left and right masseter muscles in oral muscles and ratios of the amounts of work of suprahyoid muscles measured during mastication with respect to the amounts of work of the oral muscles (the left and right masseter muscles and the suprahyoid muscles) required for mastication, where (A) illustrates a case of a human subject with a high eating ability, and (B) illustrates a case of a human subject with a low eating ability. The horizontal axes in Fig. 12 (A) and (B) represent the amounts of work of oral muscles required for mastication, and the vertical axes represent the amounts of work of the left and right masseter muscles and the suprahyoid muscles measured during the mastication. As illustrated in Fig. 12 (A), the human subject with the high eating ability can perform mastication up to a predetermined maximum mastication ability, and when the human subject with the high eating ability eats a mastication ability evaluation food A with a predetermined hardness, for example, the amounts of work of the left and right masseter muscles and the amounts of work of the suprahyoid muscles are indicated by the length of the part where the dashed line A crosses. When the human subject with the high eating ability eats a mastication ability evaluation food B that is harder than the mastication ability evaluation food A, the amounts of work of the left and right masseter muscles and the amounts of work of the suprahyoid muscles are indicated by the length of the part where the dashed line B crosses at a position that is different from that of the dashed line A.

As illustrated in Fig. 12 (B), the maximum mastication ability of the human subject with the low eating ability is lower than that of the human subject with the high eating ability. Also, as for the amounts of work of the left and right masseter muscles and the amounts of work of the suprahyoid muscles indicated by the length of the part where the dashed line A crosses when the mastication ability evaluation food A is taken, the ratios of the amounts of work of the left and right masseter muscles are smaller and the ratios of the amounts of work of the suprahyoid muscles are larger than those in Fig. 12 (A). As for the amounts of work of the left and right masseter muscles and the amounts of work of the suprahyoid muscles indicated by the length of the part where the dashed line B crosses, the ratios of the amounts of work of the left and right masseter muscles are smaller and the ratios of the amounts of work of the suprahyoid muscles are larger than those in Fig. 12 (A), even in the case of the mastication ability evaluation food B with a different hardness. It is considered that the ratios of the amounts of work of the left and right masseter muscles are smaller and the ratios of the amounts of work of the suprahyoid muscles are larger as the eating ability decreases for food of any hardness.

It is possible to grasp the eating movement from the beginning to the end of eating by using the eating ability evaluation method according to the present embodiment. In a case where a curry rice set including curry rice, fukujinzuke pickles, green bean salad, and canned peaches is eaten, various sizes and physical properties of curry roux with high viscosity, hard potatoes contained in it, crunchy fukujinzuke pickles, fibrous green beans, peaches that require biting with front teeth to adjust the amount of one bite, and the like are included. In a case where a person with a high eating ability eats this, the person can eat it at his/her own pace while adjusting the food to the amount of one bite without being affected by differences in physical properties and while enjoying various textures. In other words, masseter muscles are dominantly used for mastication, and the ratios of the amounts of work of the masseter muscles and the suprahyoid muscles are stable. On the other hand, a person with a low mastication ability cannot address the differences in physical properties of the ingredients, and may eat rice separately from curry roux, leave fukujinzuke pickles, chop potatoes into small pieces and eat them separately from the curry roux and rice, chew green beans several times and swallow them with water, for example, and eating time may become long, or the person may get tired and leave a lot of food. In other words, the ratios of the amounts of work of the left and right masseter muscles and the suprahyoid muscles are unstable, and the ratios of the amounts of work of the masseter muscles are low for ingredients that are difficult to be crunched with teeth by using the left and right masseter muscles. As an exception, the ratios of the amounts of work of the masseter muscles and the suprahyoid muscles are less likely to be affected by the mastication ability for noodles that are to be slurped and eaten, hiyayakko tofu that can be collapsed with a tongue, soup, and the like.

As described above, the above eating ability evaluation method by which it is possible to compare mastication motions when a plurality of kinds of food is eaten and to monitor mastication motions when food is successively eaten can reflect the mastication ability in one meal, which cannot be completely grasped on the basis of how large the amounts of work when one food is eaten are.

In the above embodiments and modification examples, various modifications can be made as follows.

Although which of the left and right masseter muscles and the suprahyoid muscles dominantly contribute to mastication is determined and the eating ability is evaluated on the basis of the ratios of the amounts of work of the left and right masseter muscles and the amounts of work of the suprahyoid muscles in the above embodiments, the evaluation device is not limited to the above description. For example, either the determination of which of the left and right masseter muscles and the suprahyoid muscles dominantly contribute to the mastication or the evaluation of eating ability may be performed. Alternatively, the device may not perform both of them and is adapted only to output the ratios of the amounts of work of the muscles in this case. For example, how high the eating ability is may be categorized into two levels, namely a high level and a low level, or into multiple levels other than three levels.

### <Example 7>

Example 7 will be described. Example 7 is an example related to ingredients configuring food that a human subject eats.

In another example, the evaluation device 1 measures myoelectric potential data when a human subject masticates a certain food, for example, and evaluates an eating ability of the human subject on the basis of the measured myoelectric potential data. In Example 7, what kinds of ingredients the food to be taken were preferably configured by in evaluation in an eating ability was examined. Note that the following examination was assumed to be performed by a test executer.

The test executer conducted an experiment using a "pork and tofu set" as food, for example. The food includes a single dish or a set of dishes. For example, the "pork and tofu set" is a meal set including "porridge" as a staple food and "tofu" and the like as side dishes in addition to a main dish ingredient ("fried pork"), and that is, a meal set including pork and tofu. Also, the "pork and tofu set" is assumed to be similarly called "pork and tofu set" even in a case where some of the ingredients were excluded in the following experiments and "pork" and "tofu" were not included.

Fig. 13 is a diagram illustrating an example of the ingredients included in the "pork and tofu set". In Fig. 13, the left sections indicate names of the ingredients, and the right sections indicate hardnesses of the ingredients using a unit of pressure (N/cm²: newton per square centimeter). The pressure is, for example, a pressure at which the ingredients can be smushed (or the ingredients start to be smushed). Note that the hardness in Fig. 13 is, for example, a value obtained by performing measurement on each ingredient extracted after cooking.

In the following experiment, the ingredients were categorized into three levels in accordance with hardnesses. The first level corresponds to the softest ingredients, and the ingredients of less than 1 N/cm² will be called "ingredients that can be processed with a tongue". The second level corresponds to ingredients that are soft to such an extent that mastication was needed, and ingredients of not less than 1 N/cm² and less than 30 N/cm² will be called "soft ingredients". The third level corresponds to the hardest ingredients, and ingredients of not less than 30 N/cm² will be called "hard ingredients".

### <1. Experiment procedure>

Hereinafter, an experiment procedure will be described. In the experiment, the following processes are performed, for example.

### <1.1 Food preparation process>

A food preparation process was a process of preparing (getting ready for) food "pork and tofu". The test executer prepared "pork and tofu" in four patterns for each ingredients included in the food.
Food pattern 1: All the ingredients were included.
Food pattern 2: "Ingredients that were able to be processed with a tongue" were not included.
Food pattern 3: "Soft ingredients" were not included.
Food pattern 4: "Hard ingredients" were not included.

The test executer prepared "pork and tofu" in the food pattern 1 first. Then, the test executer prepared "pork and tofu" in the food patterns 2 to 4 by excluding the ingredients in each level illustrated in Fig. 13 from "pork and tofu" in the food pattern 1.

### <1.2 Myoelectric potential measurement process>

A myoelectric potential measurement process was a process of measuring a myoelectric potential during mastication of a human subject. Measurement locations were, for example, left and right masseter muscles and suprahyoid muscles similarly to Example 1, for example. The test executer prepared one or more human subjects with high eating abilities, one or more human subjects with middle eating abilities, and one or more human subjects with low eating abilities, for example. Note that the eating abilities were assumed to be similar to those in the aforementioned examples, for example.

The test executer asked each human subject to eat the "pork and tofu" in the food patterns 1 to 4 and measured myoelectric potentials during mastication. The measurement of the myoelectric potentials was performed by the evaluation device 1, for example. The test executer acquired myoelectric potential data of measurement target muscles in combinations of the human subjects with each eating abilities and the food patterns.

Also, the test executer captured videos of the human subjects in synchronization with the measurement of the myoelectric potentials when the human subjects ate the food. The test executer was able to recognize motions other than eating of the human subjects and exclude noise data from the myoelectric potential data through analysis of the captured video data. Note that it was possible to use any data to be analyzed (motion analysis data) as long as motions of the human subjects during the mastication were able to be determined and recorded sound data obtained by recording sound around the human subjects (around their mouths), for example, was able to be used.

Note that the myoelectric potential measurement process was able to be performed by a test executer or a device that was different from the test executer and the evaluation device to perform the myoelectric potential data analysis process, which will be described below. For example, the test executer was able to acquire myoelectric potential data measured at a different location or by a different device to use the myoelectric potential data for the analysis. Alternatively, the test executer was able to acquire myoelectric potential data by receiving data measured within Japan or in a foreign country, for example, through communication via the Internet.

### <1.3 Myoelectric potential data analysis process>

A myoelectric potential data analysis process was a process of calculating a feature amount of the myoelectric potential data for each of the eating abilities of the human subjects or for each food pattern. The test executer sectioned the measured myoelectric potential data for each unit time and calculated ratios of the amounts of work of the muscles. The unit time included, for example, a time determined in advance and a time to perform a series of mastication motions (for example, a time to perform mastication motions for one bite or a time to perform motions from the start of eating to the end of eating of entire (a predetermined amount of) food). The amounts of work were numerical values indicating how much certain muscles had moved and were, for example, myoelectric potentials (measured values). The ratios of the amounts of work were proportions of the amounts of work of certain muscles (first muscles) with respect to a total value of the amounts of work of all the muscles subjected to the measurement and were represented by, for example, percentages (%). The amounts of work was able to be the amounts of work in other examples.

The test executer determined whether or not the human subjects were eating targets ("pork and tofu" in this case) from the captured video, determined motions from the timing at which each human subject put ingredients into his/her mouth and started mastication (this was determined from characteristic myoelectric potentials of masseter muscles, for example) to the timing immediately before swallowing them (this was determined from characteristic myoelectric potentials from suprahyoid muscles, for example) as a series of mastication motions, and extracted myoelectric potential data as analysis targets.

The test executer excluded the following myoelectric potential data as noise data by further analyzing the captured video.
- When the human subjects ate a plurality of kinds of food at the same time (rice and pork, for example)
- When the human subjects ate some food while drinking something (rice and tea, for example)
- When the human subjects did not chew anything (because myoelectric potentials were measured regardless of the human subjects not masticating)
- When the human subjects had conversation during meal (because myoelectric potentials were measured regardless of the human subjects not masticating; conversation made while masticating was also excluded)
- Other motions such as wiping off their mouths (because myoelectric potentials were measured regardless of the human subjects not masticating)

The test executer categorized the extracted myoelectric potential data for each combination of a mastication ability and a food pattern and calculated feature amounts to be analyzed. The feature amounts to be analyzed were, for example, ranges and medians. Note that the test executer was able to use myoelectric potential data of one human subject or was able to use myoelectric potential data of the same number of human subjects for each eating ability in the calculation of the feature amounts in a case where there were a plurality of human subjects with each mastication ability.

### <2. Experiment results>

Hereinafter, experiment results will be described. Experiment results will be described for each food pattern. Note that in the experiment, the ratios of the amounts of work were ratios of the amounts of work of left and right masseter muscles with respect to the entire amount of work. Also, it was assumed that the number of human subjects with each mastication ability was one in the experiment.

### <2.1 Food pattern 1>

Fig. 14 is a diagram illustrating an example of the ratios of the amounts of work of the masseter muscles in regard to the food pattern 1 (including all the ingredients). In the graph in Fig. 14, the vertical axis represents the ratio of the amount of work, and the horizontal axis represents the eating ability of each human subject. Also, "Me" denotes a median of the ratios of the amounts of work, and "R" denotes a range of the ratios of the amounts of work in Fig. 14. The same applies to the graphs in the following drawings.

According to Fig. 14, the range and the median for the human subject with the high eating ability were 57.0 and 55.1%, respectively. The range and the median for the human subject with the middle eating ability were 28.3 and 43.8%, respectively. The range and the median for the human subject with the low eating ability were 25.0 and 25.4%, respectively.

The range was wider as the eating ability was higher in the myoelectric potential measurement using the food pattern 1. It was possible to assume that this phenomenon was attributable to the fact that persons with higher eating abilities changed how to masticate to higher extent in accordance with hardnesses of the food (ingredients).

Also, the median was higher as the eating ability was higher in the myoelectric potential measurement using the food pattern 1. It was possible to assume that this phenomenon was attributable to the fact that persons with higher eating ability more frequently used masseter muscles (performed more chewing motions).

### <2.2 Food pattern 2>

Fig. 15 is a diagram illustrating an example of ratios of the amounts of work of the masseter muscles in regard to the food pattern 2 (excluding the ingredients that can be processed by tongues).

According to Fig. 15, the range and the median for the human subject with the high eating ability were 55.0 and 55.4%, respectively. The range and the median for the human subject with the middle eating ability were 24.9 and 45.9%. The range and the median for the human subject with the low eating ability were 25.0 and 37.7%, respectively.

The median was higher as the eating ability was higher in the myoelectric potential measurement using the food pattern 2.

However, although the range was the widest for the human subject with the high eating ability, the range for the human subject with the middle eating ability was lower than that for the human subject with the low eating ability, in the myoelectric potential measurement using the food pattern 2. In other words, it was not possible to state that the range was always higher as the eating ability was higher.

### <2.3 Food pattern 3>

Fig. 16 is a diagram illustrating an example of ratios of the amounts of work of the masseter muscles in regard to the food pattern 3 (excluding the soft ingredients).

According to Fig. 16, the range and the median for the human subject with the high eating ability were 54.2 and 66.7%, respectively. The range and the median for the human subject with the middle eating ability were 28.3 and 44.0%, respectively, the range and the median for the human subject with the low eating ability were 25.0 and 33.1%, respectively.

The range was wider as the eating ability was higher in the myoelectric potential measurement using the food pattern 3.

The median was higher as the eating ability was higher in the myoelectric potential measurement using the food pattern 3.

### <2.4 Food pattern 4>

Fig. 17 is a diagram illustrating an example of ratios of the amounts of work of the masseter muscles in regard to the food pattern 4 (excluding the hard ingredients).

According to Fig. 17, the range and the median for the human subject with the high eating ability were 57.0 and 52.4%, respectively. The range and the median for the human subject with the middle eating ability were 23.7 and 40.4%, respectively. The range and the median for the human subject with the low eating ability were 24.5 and 35.8%, respectively.

The median was higher as the eating ability was higher in the myoelectric potential measurement using the food pattern 4.

However, although the range was the widest for the human subject with the high eating ability, the range of the human subject with the middle eating ability was narrower than that of the human subject with the low eating ability, in the myoelectric potential measurement using the food pattern 4. In other words, a relationship in which the range was wider as the eating ability was higher was not always achieved.

### <2.5 Comparison for each food pattern>

In a case where the food patterns 2 and 4 were used, the median was higher as the eating ability was higher. However, the relationship in which the range was wider as the eating ability was higher was not always achieved. Although it was possible to perform evaluation based on the median, the evaluation based on the range was not correct in some cases in the case where the food patterns 2 and 4 were used.

On the other hand, in a case where the food patterns 1 and 3 were used, the median was higher as the eating ability was higher, and the range was wider as the eating ability was higher. In other words, it was possible to perform evaluation using both the criteria, namely the median and the range in the case where the food patterns 1 and 3 were used, and it was considered to be possible to perform accurate evaluation as compared with the case where the food patterns 2 and 4 were used.

The food pattern 1 and the food pattern 3 will be compared and examined. Hereinafter, differences between the values of the human subject with the high eating ability and the values of the human subject with the middle eating ability and differences between the values of the human subject with the middle eating ability and the values of the human subject with the low eating ability will be compared and examined.

As for the food pattern 1, a difference between the range for the human subject with the high eating ability and the range for the human subject with the middle eating ability was 28.7 (57.0 - 28.3), and a difference between the range for the human subject with the middle eating ability and the range for the human subject with the low eating ability was 3.3 (28.3 - 25.0). On the other hand, as for the food pattern 3, a difference between the range for the human subject with the high eating ability and the range for the human subject with the middle eating ability was 25.9 (54.2 - 28.3), and a difference between the range for the human subject with the middle eating ability and the range for the human subject with the low eating ability was 3.3 (28.3 - 25.0). Both the food patterns showed similar numerical values as the differences in ranges. In other words, in the case where the food pattern 1 and the food pattern 3 were used, there were substantially no differences in evaluation based on the ranges, it was possible to perform equivalent evaluation.

Also, as for the food pattern 1, a difference between the median for the human subject with the high eating ability and the median for the human subject with the middle eating ability was 11.3 (55.1 - 43.8), and a difference between the median for the human subject with the middle eating ability and the median for the human subject with the low eating ability was 8.4 (43.8 - 35.4). On the other hand, as for the food pattern 3, a difference between the median for the human subject with the high eating ability and the median for the human subject with the middle eating ability was 22.7 (66.7 - 44.0), and a difference between the median for the human subject with the middle eating ability and the median for the human subject with the low eating ability was 10.9 (44.0 - 33.1). The differences in medians slightly clearly appeared in the case where the food pattern 3 was used, particularly between the human subject with the high eating ability and the human subject with the middle eating ability. In other words, it was possible to assume that the food pattern 3 was able to be used to perform evaluation with accuracy that was equivalent to or more than the accuracy achieved by the food pattern 1 in the evaluation based on the medians.

As a summary of the comparison and examination results, it was possible to perform the evaluation using the medians and the ranges with the food pattern 1 and the food pattern 3 and thereby to state that the food pattern 1 and the food pattern 3 were more excellent than the food pattern 2 and the food pattern 4. On the other hand, since the food pattern 3 brought about differences that were equivalent to or more significant than those achieved by the food pattern 1, it was possible to state that the food pattern 3 was more excellent than the food pattern 1.

### <Example 8>

Example 8 will be described. In Example 8, muscles from which myoelectric potentials are measured are changed in another example.

In another example, left and right masseter muscles and suprahyoid muscles are measurement targets. The measurement targets may be zygomatic muscles (one of or both zygomaticus major muscles and zygomaticus minor muscles) and suprahyoid muscles. Also, the measurement targets may be temporalis muscles and suprahyoid muscles. Furthermore, the measurement targets may be a masseter muscle and a suprahyoid muscle on either left or right side. The zygomatic muscles and the temporalis muscles are muscles involved in mastication of a human, and myoelectric potential data therefrom indicates features similar to those of the masseter muscles. Also, since differences between the masseter muscles on the right side and the left side are small (similar trends are shown), measuring one of them may be sufficient.

Also, the measurement targets may be a plurality of combinations of left and right masseter muscles, zygomatic muscles, and temporalis muscles in addition to the suprahyoid muscles. Since features indicated by the zygomatic muscles and the temporalis muscles are not completely the same although they indicate similar features, evaluation accuracy is improved by combining a plurality of muscles involved in mastication.

### <Example 9>

For example, a first threshold value to distinguish a human subject with a high eating ability and a human subject with a middle eating ability and a second threshold value to distinguish the human subject with the middle eating ability and a human subject with a low eating ability were set for one of or both a range and a median, and eating abilities of the human subjects were evaluated in Example 9. Many pieces of experiment data was acquired, and the first and second threshold values were set in accordance with the acquired data. The setting of the threshold values and the evaluation may be performed by an evaluation artificial intelligence (AI) (hereinafter, referred to as an evaluation model), for example.

The evaluation model performs learning using training data including feature amounts (for example, the median and the range; an average value, a mode, or the like may also be included) of myoelectric potential data and eating abilities (for example, high, middle, low, or the like; scored values may also be used) of human subjects, for example. Note that the myoelectric potential data itself may also be used instead of the feature amounts of the myoelectric potential data. In this case, the feature amounts of the myoelectric potential data are calculated in internal processing of a threshold value generation model.

Once determination target data including the myoelectric potential data or the feature amounts of the myoelectric potential data is input, the threshold value generation model after the learning generates the first threshold value and the second threshold value in the internal processing, for example, and outputs eating abilities (high, middle, or low; or a score) of the human subjects of the determination target data.

For the learning of the evaluation model, evaluation results in actual operations, for example, may be used. In this manner, the training data increases and evaluation accuracy of the evaluation model is further improved as more operations are performed.

Also, since using of the evaluation model makes it easy to increase or decrease the kinds of feature amounts, calculate the feature amounts, and calculate the threshold values, it is possible to reduce the workload of a test executer.

Note that although the aforementioned myoelectric potential data was data in mastication of the "pork and tofu set", myoelectric potential data may be acquired using a plurality of kinds of food or meal sets, for example. Fig. 18 is a diagram illustrating an example of ingredients included in certain meal sets. Fig. 18 illustrates examples of ingredients included in a "pork and tofu set", a "curry set", a "somen noodle set", and a "pork and broccoli set".

The human subjects may have these meals on a plurality of days or a plurality of times, for example, the human subjects may eat the "pork and tofu sets" on the first day, eat the "curry sets" on the second day, eat the "somen noodle sets" on the third day, and eat the "pork and broccoli sets" on the fourth day, for example. Each meal set to be used for the experiment may be configured of a combination of a single food and a meal set, for example. The ingredients with each hardness are not necessarily included in food or a meal set to be taken once and may be taken a plurality of number of times in meal sets as illustrated in Fig. 18.

### REFERENCE SIGNS LIST

1: EVALUATION DEVICE
11: FIRST MYOELECTRIC MEASUREMENT UNITS
12: SECOND MYOELECTRIC MEASUREMENT UNITS
13: SWALLOWING MEASUREMENT UNIT
14: SIGNAL PROCESSING UNIT
21: ELECTROMYOGRAM ACQUISITION UNIT 42
22: LEFT AND RIGHT MASSETER MUSCLES WORK AMOUNT ACQUISITION UNIT
23: SUPRAHYOID MUSCLES WORK AMOUNT ACQUISITION UNIT
24: LEFT AND RIGHT MASSETER MUSCLES AND SUPRAHYOID MUSCLES WORK AMOUNT RATIO ACQUISITION UNIT
25: DETERMINATION UNIT
26: EVALUATION UNIT
35: STORAGE UNIT

## Claims

1. An eating ability evaluation method comprising:
an acquisition step of acquiring myoelectric potential data on a plurality of facial muscles when a subject person masticates;
a calculation step of calculating a ratio of an amount of work of a first muscle, which is one of the plurality of facial muscles involved in mastication, to a total amount of work of all of the plurality of facial muscles involved in mastication for each unit time on the basis of the acquired myoelectric potential data; and
an evaluation step of evaluating an eating ability of the subject person based on the ratio.

2. The eating ability evaluation method according to claim 1,
wherein the plurality of muscles include a masseter muscle and suprahyoid muscles, and
the first muscle is the masseter muscle.

3. The eating ability evaluation method according to claim 1, wherein the unit time is from a start of mastication to a timing immediately before swallowing.

4. The eating ability evaluation method according to claim 1,
wherein in the acquisition step, motion analysis data for analyzing motion of the subject person when the myoelectric potential data is measured is acquired, and
in the calculation step, the myoelectric potential data in a period during which the subject person is not masticating in the motion analysis data is excluded from a calculation target of the ratio.

5. The eating ability evaluation method according to claim 1, wherein in the evaluation step, the eating ability is determined to be higher as a range between a maximum value and a minimum value of the ratio in a predetermined time becomes wider.

6. The eating ability evaluation method according to claim 1, wherein in the evaluation step, the eating ability is evaluated in accordance with a median and a range between a maximum value and a minimum value of the ratio in a predetermined time.

7. The eating ability evaluation method according to claim 1, wherein the myoelectric potential data is a value measured when the subject person masticates food that contains all of an ingredient with a first hardness, an ingredient with a second hardness that is higher than the first hardness, and an ingredient with a third hardness that is higher than the ingredient with the second hardness.

8. The eating ability evaluation method according to claim 1, wherein the myoelectric potential data is a value measured when the subject person masticates food that, from among an ingredient with a first hardness, an ingredient with a second hardness that is higher than the first hardness, and an ingredient with a third hardness that is higher than the ingredient with the second hardness, contains the ingredients with the first hardness and the third hardness and does not contain the ingredient with the second hardness.

9. The eating ability evaluation method according to claim 8,
wherein the first hardness food is a food that can be crushed by a pressure of less than 1 N/cm²,
the second hardness food is a food that can be crushed by a pressure of 1 N/cm² or more and less than 30 N/cm², and
the third hardness food is a food that can be crushed by a pressure of 30 N/cm² or more.

10. The eating ability evaluation method according to claim 1,
wherein the plurality of muscles include left and right masseter muscles and suprahyoid muscles, and
the amount of work of the first muscle is a total value of amounts of work of the left and right masseter muscles.

11. The eating ability evaluation method according to claim 1,
wherein the plurality of muscles is a combination of one or more of left and right masseter muscles, and temporalis muscles in addition to suprahyoid muscles, and
the amount of work of the first muscle is a total value of an amount of work of one kind of the left and right masseter muscles, and temporalis muscles or an amount of work of a combination thereof.

12. The eating ability evaluation method according to claim 1,
wherein the evaluation step is performed using an evaluation model,
the evaluation model is
a model performing learning using myoelectric potential data for learning and eating ability data of a measuring person who has measured the myoelectric potential data for learning and generating a threshold value in the learning, and
in the evaluation step, the eating ability of the subject person is output by inputting the ratio and comparing the generated threshold value with the ratio.

13. An eating ability evaluation device (1) comprising:
an acquirer (21) that acquires myoelectric potential data of a plurality of facial muscles when a subject person masticates;
a calculator (24) that calculates a ratio of an amount of work of a first muscle, which is one of the plurality of facial muscles involved in mastication, to a total amount of work of all of the plurality of facial muscles involved in mastication on the basis of the acquired myoelectric potential data; and
an evaluator (26) that evaluates an eating ability of the subject person based on the ratio.

## Patentansprüche

1. Essfähigkeitsevaluierungsverfahren, umfassend:
einen Erfassungsschritt zum Erfassen myoelektrischer Potenzialdaten über eine Vielzahl von Gesichtsmuskeln, wenn eine Versuchsperson kaut;
einen Berechnungsschritt zum Berechnen eines Verhältnisses einer Arbeitsmenge eines ersten Muskels, der einer aus der Vielzahl von Gesichtsmuskeln, die am Kauen beteiligt sind, ist, zu einer Gesamtarbeitsmenge aller aus der Vielzahl von Gesichtsmuskeln, die am Kauen beteiligt sind, für jede Zeiteinheit auf der Grundlage der erfassten myoelektrischen Potenzialdaten; und
einen Evaluierungsschritt zum Evaluieren einer Essfähigkeit der Versuchsperson basierend auf dem Verhältnis.

2. Essfähigkeitsevaluierungsverfahren nach Anspruch 1,
wobei die Vielzahl von Muskeln einen Massetermuskel und suprahyoidale Muskeln enthält, und
der erste Muskel der Massetermuskel ist.

3. Essfähigkeitsevaluierungsverfahren nach Anspruch 1, wobei sich die Zeiteinheit von einem Beginn des Kauens bis zu einem Zeitpunkt unmittelbar vor dem Schlucken erstreckt.

4. Essfähigkeitsevaluierungsverfahren nach Anspruch 1,
wobei in dem Erfassungsschritt Bewegungsanalysedaten zum Analysieren einer Bewegung der Versuchsperson, wenn die myoelektrischen Potenzialdaten gemessen werden, erfasst werden und
in dem Berechnungsschritt die myoelektrischen Potenzialdaten in einem Zeitraum, in dem die Versuchsperson gemäß den Bewegungsanalysedaten nicht kaut, von einer Berechnung des Verhältnisses ausgeschlossen werden.

5. Essfähigkeitsevaluierungsverfahren nach Anspruch 1, wobei in dem Evaluierungsschritt die Essfähigkeit als umso höher bestimmt wird, je größer ein Bereich zwischen einem Maximalwert und einem Minimalwert des Verhältnisses in einer vorbestimmten Zeit wird.

6. Essfähigkeitsevaluierungsverfahren nach Anspruch 1, wobei in dem Evaluierungsschritt die Essfähigkeit entsprechend einem Median und einem Bereich zwischen einem Maximalwert und einem Minimalwert des Verhältnisses in einer vorbestimmten Zeit evaluiert wird.

7. Essfähigkeitsevaluierungsverfahren nach Anspruch 1, wobei die myoelektrischen Potenzialdaten einen Wert darstellen, der gemessen wird, wenn die Versuchsperson Nahrung kaut, die eine Zutat mit einer ersten Härte, eine Zutat mit einer zweiten Härte, die höher als die erste Härte ist, und eine Zutat mit einer dritten Härte, die höher als die Härte der Zutat mit der zweiten Härte ist, enthält.

8. Essfähigkeitsevaluierungsverfahren nach Anspruch 1, wobei die myoelektrischen Potenzialdaten einen Wert darstellen, der gemessen wird, wenn die Versuchsperson Nahrung kaut, die von einer Zutat mit einer ersten Härte, einer Zutat mit einer zweiten Härte, die höher als die erste Härte ist, und einer Zutat mit einer dritten Härte, die höher als die Härte der Zutat mit der zweiten Härte ist, die Zutaten mit der ersten Härte und der dritten Härte enthält und die Zutat mit der zweiten Härte nicht enthält.

9. Essfähigkeitsevaluierungsverfahren nach Anspruch 8,
wobei die Nahrung mit der ersten Härte eine Nahrung ist, die durch einen Druck von weniger als 1 N/cm² zerdrückt werden kann,
die Nahrung mit der zweiten Härte eine Nahrung ist, die durch einen Druck von 1 N/cm² oder mehr und weniger als 30 N/cm² zerdrückt werden kann, und
die Nahrung mit der dritten Härte eine Nahrung ist, die durch einen Druck von 30 N/cm² oder mehr zerdrückt werden kann.

10. Essfähigkeitsevaluierungsverfahren nach Anspruch 1,
wobei die Vielzahl von Muskeln linke und rechte Massetermuskeln und suprahyoidale Muskeln enthält, und
die Arbeitsmenge des ersten Muskels ein Gesamtwert von Arbeitsmengen der linken und rechten Massetermuskeln ist.

11. Essfähigkeitsevaluierungsverfahren nach Anspruch 1,
wobei die Vielzahl von Muskeln eine Kombination aus einem oder mehreren der linken und rechten Massetermuskeln und Temporalismuskeln zusätzlich zu suprahyoidalen Muskeln ist, und
die Arbeitsmenge des ersten Muskels ein Gesamtwert einer Arbeitsmenge einer Art der linken und rechten Massetermuskeln und Temporalismuskeln oder einer Arbeitsmenge einer Kombination davon ist.

12. Essfähigkeitsevaluierungsverfahren nach Anspruch 1,
wobei der Evaluierungsschritt unter Verwendung eines Evaluierungsmodells durchgeführt wird,
das Evaluierungsmodell ein Modell ist, das unter Verwendung von myoelektrischen Potenzialdaten zum Lernen und Essfähigkeitsdaten einer Messperson, die die myoelektrischen Potenzialdaten zum Lernen gemessen hat, ein Lernen durchführt und bei dem Lernen einen Schwellenwert erzeugt, und
in dem Evaluierungsschritt die Essfähigkeit der Versuchsperson ausgegeben wird, indem das Verhältnis eingegeben und der erzeugte Schwellenwert mit dem Verhältnis verglichen wird.

13. Essfähigkeitsevaluierungsvorrichtung (1), umfassend:
eine Erfassungseinrichtung (21), die myoelektrische Potenzialdaten einer Vielzahl von Gesichtsmuskeln, wenn eine Versuchsperson kaut, erfasst;
eine Berechnungseinrichtung (24), die ein Verhältnis einer Arbeitsmenge eines ersten Muskels, der einer aus der Vielzahl von Gesichtsmuskeln, die am Kauen beteiligt sind, ist, zu einer Gesamtarbeitsmenge aller aus der Vielzahl von Gesichtsmuskeln, die am Kauen beteiligt sind, auf der Grundlage der erfassten myoelektrischen Potenzialdaten berechnet; und
eine Evaluierungseinrichtung (26), die eine Essfähigkeit der Versuchsperson basierend auf dem Verhältnis evaluiert.

## Revendications

1. Procédé d'évaluation de la capacité d'alimentation comprenant :
une étape d'acquisition consistant à acquérir des données de potentiel myoélectrique sur une pluralité de muscles faciaux lorsqu'une personne sujet mastique ;
une étape de calcul consistant à calculer un rapport entre une quantité de travail d'un premier muscle, qui est l'un de la pluralité de muscles faciaux impliqués dans la mastication, et une quantité totale de travail de l'ensemble de la pluralité de muscles faciaux impliqués dans la mastication pour chaque unité de temps sur la base des données de potentiel myoélectrique acquises ; et
une étape d'évaluation consistant à évaluer une capacité d'alimentation de la personne sujet sur la base du rapport.

2. Procédé d'évaluation de la capacité d'alimentation selon la revendication 1,
la pluralité de muscles comprenant un muscle masséter et des muscles suprahyoïdes, et
le premier muscle étant le muscle masséter.

3. Procédé d'évaluation de la capacité d'alimentation selon la revendication 1, l'unité de temps s'étendant d'un début de mastication à un moment immédiatement avant la déglutition.

4. Procédé d'évaluation de la capacité d'alimentation selon la revendication 1,
dans l'étape d'acquisition, des données d'analyse de mouvement pour analyser le mouvement de la personne sujet lorsque les données de potentiel myoélectrique sont mesurées étant acquises, et
dans l'étape de calcul, les données de potentiel myoélectrique dans une période pendant laquelle la personne sujet ne mastique pas dans les données d'analyse de mouvement étant exclues d'une cible de calcul du rapport.

5. Procédé d'évaluation de la capacité d'alimentation selon la revendication 1, dans l'étape d'évaluation, la capacité d'alimentation étant déterminée comme étant plus élevée lorsqu'une plage entre une valeur maximale et une valeur minimale du rapport dans un temps prédéterminé devient plus large.

6. Procédé d'évaluation de la capacité d'alimentation selon la revendication 1, dans l'étape d'évaluation, la capacité d'alimentation étant évaluée en fonction d'une médiane et d'une plage entre une valeur maximale et une valeur minimale du rapport dans un temps prédéterminé.

7. Procédé d'évaluation de la capacité d'alimentation selon la revendication 1, les données de potentiel myoélectrique étant une valeur mesurée lorsque la personne sujet mastique un aliment qui contient à la fois un ingrédient ayant une première dureté, un ingrédient ayant une deuxième dureté qui est supérieure à la première dureté, et un ingrédient ayant une troisième dureté qui est supérieure à l'ingrédient ayant la deuxième dureté.

8. Procédé d'évaluation de la capacité d'alimentation selon la revendication 1, les données de potentiel myoélectrique étant une valeur mesurée lorsque la personne sujet mastique un aliment qui, parmi un ingrédient ayant une première dureté, un ingrédient ayant une deuxième dureté qui est supérieure à la première dureté, et un ingrédient ayant une troisième dureté qui est supérieure à l'ingrédient ayant la deuxième dureté, contient les ingrédients ayant la première dureté et la troisième dureté et ne contient pas l'ingrédient ayant la deuxième dureté.

9. Procédé d'évaluation de la capacité d'alimentation selon la revendication 8,
l'aliment de première dureté étant un aliment qui peut être écrasé par une pression inférieure à 1 N/cm²,
l'aliment de deuxième dureté étant un aliment qui peut être écrasé par une pression de 1 N/cm² ou plus et inférieure à 30 N/cm², et
l'aliment de troisième dureté étant un aliment qui peut être écrasé par une pression de 30 N/cm² ou plus.

10. Procédé d'évaluation de la capacité d'alimentation selon la revendication 1,
la pluralité de muscles comprenant des muscles masséters gauche et droit et des muscles suprahyoïdes, et
la quantité de travail du premier muscle étant une valeur totale des quantités de travail des muscles masséters gauche et droit.

11. Procédé d'évaluation de la capacité d'alimentation selon la revendication 1,
la pluralité de muscles étant une combinaison d'un ou plusieurs des muscles masséters gauche et droit, et des muscles temporaux en plus des muscles suprahyoïdes, et
la quantité de travail du premier muscle étant une valeur totale d'une quantité de travail d'un type parmi les muscles masséters gauche et droit, et les muscles temporaux, ou d'une quantité de travail d'une combinaison de ceux-ci.

12. Procédé d'évaluation de la capacité d'alimentation selon la revendication 1,
l'étape d'évaluation étant réalisée en utilisant un modèle d'évaluation, le modèle d'évaluation étant
un modèle effectuant un apprentissage à l'aide de données de potentiel myoélectrique pour l'apprentissage et de données de capacité d'alimentation d'une personne de mesure qui a mesuré les données de potentiel myoélectrique pour l'apprentissage, et générant une valeur seuil dans l'apprentissage, et
au cours de l'étape d'évaluation, la capacité d'alimentation de la personne sujet étant émise par entrée du rapport et comparaison de la valeur seuil générée avec le rapport.

13. Dispositif (1) d'évaluation de la capacité d'alimentation comprenant :
un module d'acquisition (21) qui acquiert des données de potentiel myoélectrique d'une pluralité de muscles faciaux lorsqu'une personne sujet mastique ;
un module de calcul (24) qui calcule un rapport entre une quantité de travail d'un premier muscle, qui est l'un de la pluralité de muscles faciaux impliqués dans la mastication, et une quantité totale de travail de l'ensemble de la pluralité de muscles faciaux impliqués dans la mastication sur la base des données de potentiel myoélectrique acquises ; et
un module d'évaluation (26) qui évalue une capacité d'alimentation de la personne sujet sur la base du rapport.
